# EUROPEAN PATENT APPLICATION

(11) **EP 4 079 753 A1**
(43) Date of publication of application: **26.10.2022**
(21) Application number: 21169356.9
(22) Date of filing: 20.04.2021
(51) Int. Cl.: C07K 14/47, A61K 35/17, A61P 35/00, C07K 14/725, C12N 5/0783

(54) **T-CELL RECEPTORS SPECIFIC FOR BOTH RAC1- AND RAC2-DERIVED MUTATED EPITOPES**

(71) Applicant: Max-Delbrück-Centrum für Molekulare Medizin in der Helmholtz-Gemeinschaft, 13125 Berlin (DE); Charité - Universitätsmedizin Berlin, 10117 Berlin (DE)
(72) Inventor: BLANKENSTEIN, Thomas, Berlin (DE); WILLIMSKY, Gerald, Berlin (DE)
(74) Representative: Kuttenkeuler, David

(57) **Abstract**

The invention is based on antigen binding proteins (ABPs) such as T-cell receptors (TCR) expressed on T-cells, which have a specificity to bind to MHC presented Rac2 and Rac1 derived neo-epitopes, hence such MHC presented peptides derived from mutated versions of Rac1 and Rac2, such as preferably RAC2^{P29L} and/or RAC1^{P29S}. Provided are isolated ABPs as well as genetic constructs expressing the ABPs, recombinant host cells harboring the ABP of the invention and methods for producing such ABPs and host cells. Moreover, provided are medical applications involving the TCR of the invention, for example in context of an adoptive T-cell therapy.

SEQUENCE LISTING

<110>  MAX-DELBRÜCK-CENTRUM FÜR MOLEKULARE MEDIZIN in der
       Helmholtz-Gemeinschaft
       Charité - Universitätsmedizin Berlin

<120>  T-CELL RECEPTORS SPECIFIC FOR BOTH RAC1- AND RAC2-DERIVED MUTATED
        EPITOPES

<130>  M34919EP01

<160>  18

<170>  PatentIn version 3.5

<210>  1
<211>  6
<212>  PRT
<213>  Homo sapiens

<400>  1

Asp Ser Ala Ser Asn Tyr
1               5

<210>  2
<211>  7
<212>  PRT
<213>  Homo sapiens

<400>  2

Ile Arg Ser Asn Val Gly Glu
1               5

<210>  3
<211>  14
<212>  PRT
<213>  Homo sapiens

<400>  3

Cys Ala Ala Ser Met Gly Asn Ala Gly Asn Met Leu Thr Phe
1               5                   10

<210>  4
<211>  133
<212>  PRT
<213>  Homo sapiens

<400>  4

Met Thr Ser Ile Arg Ala Val Phe Ile Phe Leu Trp Leu Gln Leu Asp
1               5                   10                  15

Leu Val Asn Gly Glu Asn Val Glu Gln His Pro Ser Thr Leu Ser Val
            20                  25                  30

Gln Glu Gly Asp Ser Ala Val Ile Lys Cys Thr Tyr Ser Asp Ser Ala
        35                  40                  45

Ser Asn Tyr Phe Pro Trp Tyr Lys Gln Glu Leu Gly Lys Gly Pro Gln
    50                  55                  60

Leu Ile Ile Asp Ile Arg Ser Asn Val Gly Glu Lys Lys Asp Gln Arg
65                  70                  75                  80

Ile Ala Val Thr Leu Asn Lys Thr Ala Lys His Phe Ser Leu His Ile
                85                  90                  95

Thr Glu Thr Gln Pro Glu Asp Ser Ala Val Tyr Phe Cys Ala Ala Ser
            100                 105                 110

Met Gly Asn Ala Gly Asn Met Leu Thr Phe Gly Gly Gly Thr Arg Leu
        115                 120                 125

Met Val Lys Pro His
    130

<210>  5
<211>  5
<212>  PRT
<213>  Homo sapiens

<400>  5

Ser Asn His Leu Tyr
1               5

<210>  6
<211>  6
<212>  PRT
<213>  Homo sapiens

<400>  6

Phe Tyr Asn Asn Glu Ile
1               5

<210>  7
<211>  15
<212>  PRT
<213>  Homo sapiens

<400>  7

Cys Ala Ser Ser Glu Thr Ser Gly Ala Ser Tyr Glu Gln Tyr Phe
1               5                   10                  15

<210>  8
<211>  134

<212>   PRT
<213>   Homo sapiens

<400>   8

Met Asp Thr Trp Leu Val Cys Trp Ala Ile Phe Ser Leu Leu Lys Ala
1               5                   10                  15

Gly Leu Thr Glu Pro Glu Val Thr Gln Thr Pro Ser His Gln Val Thr
            20                  25                  30

Gln Met Gly Gln Glu Val Ile Leu Arg Cys Val Pro Ile Ser Asn His
        35                  40                  45

Leu Tyr Phe Tyr Trp Tyr Arg Gln Ile Leu Gly Gln Lys Val Glu Phe
    50                  55                  60

Leu Val Ser Phe Tyr Asn Asn Glu Ile Ser Glu Lys Ser Glu Ile Phe
65                  70                  75                  80

Asp Asp Gln Phe Ser Val Glu Arg Pro Asp Gly Ser Asn Phe Thr Leu
                85                  90                  95

Lys Ile Arg Ser Thr Lys Leu Glu Asp Ser Ala Met Tyr Phe Cys Ala
            100                 105                 110

Ser Ser Glu Thr Ser Gly Ala Ser Tyr Glu Gln Tyr Phe Gly Pro Gly
        115                 120                 125

Thr Arg Leu Thr Val Thr
    130


<210>   9
<211>   6
<212>   PRT
<213>   Homo sapiens

<400>   9

Ser Ser Val Pro Pro Tyr
1               5


<210>   10
<211>   8
<212>   PRT
<213>   Homo sapiens

<400>   10

Tyr Thr Thr Gly Ala Thr Leu Val
1               5

<210>  11
<211>  10
<212>  PRT
<213>  Homo sapiens

<400>  11

Cys Ala Val Gly Ala Asn Asn Leu Phe Phe
1               5                   10


<210>  12
<211>  130
<212>  PRT
<213>  Homo sapiens

<400>  12

Met Leu Leu Leu Leu Val Pro Val Leu Glu Val Ile Phe Thr Leu Gly
1               5                   10                  15

Gly Thr Arg Ala Gln Ser Val Thr Gln Leu Gly Ser His Val Ser Val
            20                  25                  30

Ser Glu Gly Ala Leu Val Leu Leu Arg Cys Asn Tyr Ser Ser Ser Val
        35                  40                  45

Pro Pro Tyr Leu Phe Trp Tyr Val Gln Tyr Pro Asn Gln Gly Leu Gln
    50                  55                  60

Leu Leu Leu Lys Tyr Thr Thr Gly Ala Thr Leu Val Lys Gly Ile Asn
65                  70                  75                  80

Gly Phe Glu Ala Glu Phe Lys Lys Ser Glu Thr Ser Phe His Leu Thr
                85                  90                  95

Lys Pro Ser Ala His Met Ser Asp Ala Ala Glu Tyr Phe Cys Ala Val
            100                 105                 110

Gly Ala Asn Asn Leu Phe Phe Gly Thr Gly Thr Arg Leu Thr Val Ile
        115                 120                 125

Pro Tyr
    130


<210>  13
<211>  5
<212>  PRT
<213>  Homo sapiens

<400>  13

Ser Asn His Leu Tyr
1               5

<210>  14
<211>  6
<212>  PRT
<213>  Homo sapiens

<400>  14

Phe Tyr Asn Asn Glu Ile
1               5


<210>  15
<211>  16
<212>  PRT
<213>  Homo sapiens

<400>  15

Cys Ala Ser Ser Glu Trp Arg Gly Ser Ser Tyr Asn Glu Gln Phe Phe
1               5                   10                  15


<210>  16
<211>  135
<212>  PRT
<213>  Homo sapiens

<400>  16

Met Asp Thr Trp Leu Val Cys Trp Ala Ile Phe Ser Leu Leu Lys Ala
1               5                   10                  15

Gly Leu Thr Glu Pro Glu Val Thr Gln Thr Pro Ser His Gln Val Thr
            20                  25                  30

Gln Met Gly Gln Glu Val Ile Leu Arg Cys Val Pro Ile Ser Asn His
        35                  40                  45

Leu Tyr Phe Tyr Trp Tyr Arg Gln Ile Leu Gly Gln Lys Val Glu Phe
    50                  55                  60

Leu Val Ser Phe Tyr Asn Asn Glu Ile Ser Glu Lys Ser Glu Ile Phe
65                  70                  75                  80

Asp Asp Gln Phe Ser Val Glu Arg Pro Asp Gly Ser Asn Phe Thr Leu
                85                  90                  95

Lys Ile Arg Ser Thr Lys Leu Glu Asp Ser Ala Met Tyr Phe Cys Ala
            100                 105                 110

Ser Ser Glu Trp Arg Gly Ser Ser Tyr Asn Glu Gln Phe Phe Gly Pro
        115                 120                 125

Gly Thr Arg Leu Thr Val Leu
    130                 135

<210>  17
<211>  9
<212>  PRT
<213>  Homo sapiens

<400>  17

Phe Leu Gly Glu Tyr Ile Pro Thr Val
1               5

<210>  18
<211>  9
<212>  PRT
<213>  Homo sapiens

<400>  18

Phe Ser Gly Glu Tyr Ile Pro Thr Val
1               5

## Description

### FIELD OF THE INVENTION

The invention is based on antigen binding proteins (ABPs) such as T-cell receptors (TCR) expressed on T-cells, which have a specificity to bind to MHC presented Rac2 and Rac1 derived neo-epitopes, hence such MHC presented peptides derived from mutated versions of Rac1 and Rac2, such as preferably RAC₂^{P29L} and/or RAC1^{P29S}. Provided are isolated ABPs as well as genetic constructs expressing the ABPs, recombinant host cells harboring the ABP of the invention and methods for producing such ABPs and host cells. Moreover, provided are medical applications involving the TCR of the invention, for example in context of an adoptive T-cell therapy.

### DESCRIPTION

Human T-cells are lymphocytes that originate from bone marrow progenitor cells, and are matured in the thymus. Several T-cell subtypes are distinguished, which are categorized into CD₄+ T helper cells and CD8+ T killer cells. The different types of CD₄+ T-cells generally mediate immune functions: For example, Th1 cells are connected to immune responses against intracellular bacteria, fungi and protozoa and secrete INF-gamma and can enhance the microbial activity of macrophages. Th2 cells on the other hand play a role in the humoral immune response. By cytokine secretion and surface proteins interactions, Th2 play an important role in B-cell stimulation to secrete antibodies. CD8+ T-cells are specialized in recognizing infected or mutated cells. Upon recognition of a diseased cell, they release a mixture of perforins, granzymes and chemokines that ultimately result in apoptosis of the infected cells. (Nurieva R., Chung Y., Cellular & Molecular Immunology, 2010, 7, 190-197).

T-cell activation depends amongst others on the interaction of their unique T-cell receptors (TCR) with the specific antigen-bearing MHC receptors of antigen presenting cells. TCRs are surface anchored protein complexes that are comprised of two reactive antigen-recognizing chains and adjacent CD3 elements. In a TCR, the transmembrane CD3 protein complex mediates signal transduction via cytoplasmic ITAM-segments while the central antigen-recognizing chains interact with antigen presenting cells mainly with reactive loops on their tip.

Most commonly, the antigen interacting element of the TCR is dimerically built from two coupled TCRα and TCRβ chains. Each these TCRα or TCRβ chains is built from a constant segment with structural role and a variable segment that is responsible for antigen interaction. This interaction dominated by hypervariable regions situated at the loops of the respective amino acid chains that form the variable segments. Collectively, the combined TCRα and TCRβ variable segments form three antigen interacting complementarity determining regions (CDRs). If these are complimentary to the MHC/antigen complex of a presenting cell, a signal cascade is triggered that, depending on other co-stimulatory influences, can result in intracellular various processes or T-cell effector functions such as apoptosis of the antigen-presenting cell.

The wide range of T-cell specificity is achieved during their development in the thymus in which T-cells undergo different stages of maturation. In this process, they generate specific TCRs through somatic VJ or VDJ recombination of TCR alpha and beta encoding gene segments. Additionally, recombined TCR genes are further modified at their V-D-J and V-J gene junctions through exonuclease activity and addition of random non-template dependent nucleotides (N-nucleotides). The diversity creates highly variable complementarity determining regions that define the fine specificity of TCR chains.

TCRs recognize antigens in form of short peptide fragments that presented by cell surface glycoproteins referred to as major histocompatibility complexes (MHC) which are in humans encoded by human leukocyte antigen (HLA) genes and in mice by H-2 genes. In humans, two classes of MHC molecules are distinguished. MHC class I molecules are expressed on all nucleated cells and are recognized by CD8+ T-cells. MHC class II molecules that are expressed on professional antigen presenting cells, B-cells and activated T-cells and are recognized by CD₄+ T-cells. In MHC class I antigen presentation, intracellular proteins are endogenously processed in the proteasome into short peptide fragments that are typically 8-10 amino acids in length. After translocation across the endoplasmic reticulum through the transporter associated with antigen processing (TAP) and loading on MHC class I molecules, these antigens are presented for CD8+ T-cell recognition. Upon engagement of the TCRαβ dimer with the cognate antigen/MHC complex, the associated CD3 complex initiates the signaling cascade needed for T-cell activation. (Rath J. A., Arber C., Cells. 2020, 1485)

Adoptive T-cell therapy is a collective term for treatment procedures that rely on administering in vitro augmented T-cells with specificity towards a patient's tumor or virus-infected cells. There are different variants of ATT: For example, augmented T-cells can be generated by autologous harvesting and subsequent amplification/activation of T-cell fractions with adequate binding affinities. Other ATT therapies make use of T-cells that have been genetically engineered to either express optimized naturally-occurring TCRs, or, as in the case of CAR-T-cell therapy, to express artificially designed chimeric antigen-binding receptors. Naturally occurring TCRs off the advantage of recognizing endogenously processed extra- and intracellular antigens in an MHC-restricted manner on the cell surface and are therefore able to target a different array of antigens than CARs. T-cell based immunotherapy targets represent peptide epitopes derived from pathogen-associated or tumor-associated (or tumor-specific) proteins, which are presented by molecules of the major histocompatibility complex (MHC). These disease-associated antigens can be peptides derived from all protein classes, such as enzymes, receptors, transcription factors, etc. which are expressed and, as compared to unaltered cells of the same origin, usually up-regulated in cells of the respective tumor. In case the tumor is associated with a viral infection, such as with MCV or Human papilloma viruses (HPVs), immune therapy can be developed based on the virus antigens expressed by tumor cells which originate from virus infected host cells.

So far, ATT with TCR engineered T-cells has been tested in clinical trials mainly in patients with solid tumors expressing tumor associated or cancer-testis antigens. The first published clinical trial using TCR engineered T-cells targeted tumor associated antigens MART-i and gp100 in patients with metastatic melanoma and led to objective cancer regression rates of 19% or 30% respectively. However, the infused T-cells also recognized patient's melanocytes in the eye, ear and skin, thus leading to severe on-target off-tumor toxicity (Johnson et al. 2009). In another approach, sarcoma and melanoma patients were treated with TCR engineered T-cells targeting the cancer-testis antigen NY-ESO-1, which led to objective response rates of 55-80%, while no severe toxicities were observed (Rapoport et al. 2015 and Robbins et al. 2015). Finally, in another trial with TCR-engineered T-cells targeting cancer testis antigen MAGE-A3 in 5 out of 7 patients tumor regression was evident, but two patients died due to severe neurological toxicities (Morgan et al. 2013).

Rho GTPases of the Ras superfamily are involved in the regulation of multiple cell functions and have been implicated in the pathology of various human diseases including cancers (Fritz G., Just I., and Kaina B., Int. J. Cancer, 1999, 81, 682-687; Fritz G., Kaina B. Curr. Cancer Drug Targets, 2006, 6, 1-14; Sahai E., Marshall C.J., Nat. Rev. Cancer., 2002, 2, 133-42), pathological angiogenesis such as in diabetic retinopathy, tumoral angiogenesis, glaucoma, age-related macular degeneration (Eriksson A., Cao R., Roy J., Tritsaris K., Wahlestedt C., Dissing S., Thyberg J., Cao Y., Circulation, 2003, 107, 1532-8; Soga N., Namba N., McAllister S., Cornelius L., Teitelbaum S.L., Dowdy S.F., Kawamura J., Hruska K.A., Exp. Cell. Res., 2001, 269, 73-87; Fryer B.H., Field J., Cancer Lett., 2005, 229, 13-23), asthma, Alzheimer's disease (Desire L., Bourdin J., Loiseau N., Peillon H., Picard V., De Oliveira C., Bachelot F., Leblond B., Taverne T., Beausoleil E., Lacombe S., Drouin D., Schweighoffer F., J. Biol. Chem., 2005, 280(45), 37516-25), cardiac left ventricular hypertrophy (Brown J.H., Del Re D.P., Sussman M.A., Circ Res., 2006, 98, 730-42; Molkentin J.D., Dorn II G.W., 2nd Annu Rev Physiol. 2001, 63, 391-426). They are attractive drug targets in future targeted therapy (Nassar N., Cancelas J., Zheng J., Williams D.A., and Zheng Yi, Current topics in Medicinal Chemistry, 2006, 6, 1109-1116).

High RhoA levels have been associated with human liver, skin, colon, ovarian, bladder, gastric, esophageal squamous cell, testicular, and breast cancer. High Rho B, C, or H levels have been associated with breast, squamous cell, pancreatic, breast, liver, ovarian, head and neck, prostate, non-small cell lung, and gastric cancers and melanoma metastase. High Racl levels have been associated with human testicular, gastric, breast, and squamous cell cancers. High Rac2 or Rac3 have been associated with breast colon, head and neck, and squamous cell cancers. (Matteo Parri and Paolo Chiarugi."Rac and Rho GTPases in Cancer Cell Motility Control." Ceil Communication and Signalling. 2010(8:23). Gain-of-function mutations such as P29S of Racl were detected in melanoma, breast, head and neck cancers (Alan JK, Lundquist EA. Mutationally activated Rho GTPases in cancer. Small GTPases. 2013Jul-Sep;4(3): 159-63).

Rac proteins (Rac1, 1b, 2, 3) belong to the Rho GTP-binding proteins (or GTPases) of the Ras superfamily and thus act as molecular switches cycling between an active GTP-bound and an inactive GDP-bound form through nucleotide exchange and hydrolysis. Like most other GTPases, these proteins adopt different conformations depending on the bound nucleotide, the main differences lying in the conformation of two short and flexible loop structures designated as the switch I and switch II regions. The three distinct mammalian Rac isoforms, Rac1, 2 and 3, share a very high sequence identity (up to 90 %), with Raclb being an alternative splice variant of Rac1 with a 19 amino acid insertion in vicinity to the switch II region. Raclb has an accelerated GEF-independent GDP/GTP-exchange and an impaired GTP-hydrolysis, accounting for a self-activating GTPase (Haeusler L.C. et al., Methods in Enzymology, 2006, 406, 1-11).

RAC2 is a small GTPase belonging to the Rho family of GTPases. The RAC₂^{P29L} mutation is another so-called driver mutation facilitating tumor growth as well as metastasis and thus presents a potential target in ATT.

Thus, it is an objection of the invention to provide novel adoptive cell based immune-therapies for treating patients suffering from a cancer disease. In particular, the present invention seeks to provide novel T-cell receptor molecules that are specific to mutated RAC1- and RAC2-associated and MHC presented peptides.

### BRIEF DESCRIPTION OF THE INVENTION

Generally, and by way of brief description, the main aspects of the present invention can be described as follows:
In **a first aspect,** the invention pertains to an antigen binding protein (ABP) which, optionally, specifically binds to a RAC₂^{P29L} and/or RAC1^{P29S} associated antigenic peptide, or a variant thereof, and wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID NOs: 3, 7, 11, and 15.
In **a second aspect,** the invention pertains a nucleic acid, or a nucleic acid construct (NAC), having a nucleic acid sequence encoding for an ABP of the invention.
In **a third aspect,** the invention pertains to a recombinant vector comprising the nucleic acid of the invention.
In **a fourth aspect,** the invention pertains to a recombinant host cell, comprising or expressing an ABP of the invention, or comprising a nucleic acid or vector of the invention.
In **a fifth aspect,** the invention pertains to a pharmaceutical composition comprising one or more, preferably a combination of, an ABP of the invention, or a nucleic acid or cell of the invention.
In **a sixth aspect,** the invention pertains to a compound or composition comprising any of the products of the invention, or combinations thereof, for use in medicine, in particular for use in the treatment of a disease associated with a RAC₂^{P29L} and/or RAC1^{P29S} expression in a subject, such as cancer.
In **a seventh aspect,** the invention pertains to a method of manufacturing a RAC1/RAC2 specific antigen recognizing construct expressing cell line, comprising
   - providing a suitable host cell,
   - providing a genetic construct comprising a coding sequence encoding the ABP of the invention,
   - introducing into said suitable host cell said genetic construct,
   - expressing said genetic construct by said suitable host cell.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to an antigen binding protein (ABP) which, optionally, specifically binds to a RAC1 and/or RAC2, preferably mutated RAC1 and/or RAC2, derived antigenic peptide, or a variant thereof, and wherein the isolated ABP comprises at least one complementarity determining region (CDR) being a CDR3 having an amino acid sequence with at least 60%, 80%, 90%, 95%, 96%, 97%, 98%, or 99% sequence identity to, or having no more than three or two, preferably no more than one amino acid substitution(s), deletion(s) or insertion(s) compared to, a sequence selected from SEQ ID NOs: 3, 7, 11, and 15.

The TCR of the invention, and their respective variants, provide the surprising advantage that although a specific RAC₂^{P29L} peptide (SEQ ID NO: 17), was used, the TCR of the invention are able to further recognize RAC1^{P29S} derived peptides. This provides a surprising advantage to prior art TCR directed to such neoepitopes derived from RAC₂^{P29L} and/or RAC1^{P29S}, since when used in ATT, the constructs of the invention may treat a wider group of patients. The aspects according to the invention pertain to two principally novel HLA-A2-restricted human TCRs (RAC2-T001 and RAC2-T002) that specifically recognize the mutant protein variant Rac2^{P29L}, but also recognize the corresponding Rac variant Rac1^{P29S}. Both mutations play a role in resistance and metastatic cancers, and hence, the present invention provides novel treatment options for patients suffering from such diseases with poor prognosis.

A preferred ABP of the invention, or antigen binding variant thereof, is raised using a RAC2 derived antigenic peptide and has an increased affinity to the corresponding RAC1 antigenic peptide covering the same epitope region. Preferably, the antigenic peptide derived from RAC2 used for immunization is the peptide of SEQ ID NO: 17, whereas the ABP has an increased affinity to a peptide of SEQ ID NO: 2 derived from RAC1.

In some embodiments the ABP of the invention specifically binds to a mutated RAC1 and/or RAC2, such as RAC₂^{P29L} and/or RAC1^{P29S}, antigenic peptide-HLA molecule complex, wherein the RAC1/RAC2 antigenic peptide comprises, or alternatively consists of, a sequence of a sequence comprising the mutation preferably, a peptide sequence comprising RAC₂^{P29L} and/or RAC1^{P29S}. Such peptides are preferably 8 to 12, more preferably 10 +/-1 amino acids in length, and bind to an HLA class I (preferred) or class II molecule and/or induces T-cells cross-reacting with said peptide, or a pharmaceutically acceptable salt thereof, wherein said peptide is not the underlying full-length polypeptide. Preferably, the ABP of the invention when complexed with the antigenic peptide and the HLA induces / activates a T-cell immune response, optionally in the presence of one or more co-activating stimuli.

The term "antigen recognizing construct" or "antigen binding protein" are used herein interchangingly, and refer to proteins that have an ability to specifically bind other proteins, preferably immunoglobulin like proteins, such as T-cell receptors or antibodies or any known variants of this class of proteins.

The term "isolated" as used herein in the context of a polypeptide, such as an antigen recognizing construct (an example of which could be an antibody), refers to a polypeptide that is purified from proteins or polypeptides or other contaminants that would interfere with its therapeutic, diagnostic, prophylactic, research or other use. An antigen recognizing construct according to the invention may be a recombinant, synthetic or modified (non-natural) antigen binding construct. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. In this context, a "recombinant" protein/polypeptide or nucleic acid is one made using recombinant techniques. Methods and techniques for the production of recombinant nucleic acids and proteins are well known in the art.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site).); in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

In another additional or alternative embodiment were the ABP is preferably a TCR, the ABP may further comprise a CDR1 and/or a CDR2 domain sequence. Within the variable domain, CDR1 and CDR2 are found in the variable (V) region of a polypeptide chain, and CDR3 includes some of V, all of diversity (D) and joining (J) regions. CDR3 is the most variable and is the main CDR responsible for specifically and selectively recognizing an antigen. CDR1 and CDR2 sequences may be selected from a CDR sequence of a human variable chain allele.

Native alpha-beta heterodimeric TCRs have an alpha chain and a beta chain. Each chain comprises variable, joining and constant regions, and the beta chain also usually contains a short diversity region between the variable and joining regions, but this diversity region is often considered as part of the joining region. Each variable region comprises three CDRs (Complementarity Determining Regions) embedded in a framework sequence, one being the hypervariable region named CDR3. There are several types of alpha chain variable (Va) regions and several types of beta chain variable (Vβ) regions distinguished by their framework, CDR1 and CDR2 sequences, and by a partly defined CDR3 sequence. The Vα types are referred to in IMGT nomenclature by a unique TRAV number, Vβ types are referred to by a unique TRBV number. For more information on immunoglobulin antibody and TCR genes see the international ImMunoGeneTics information system^{®}, Lefranc M-P et al (Nucleic Acids Res. 2015 Jan;43(Database issue):D413-22; and http://www.imgt.org/).

Therefore, in one additional or alternative embodiment the ABP of the invention comprises CDR1, CDR2 and CDR3 sequences in a combination as provided in table 1 herein below, which display the respective variable chain allele together with the CDR3 sequence. Therefore, preferred are ABPs of the invention which comprise at least one, preferably, all three CDR sequences CDR1, CDR2 and CDR3. Preferably, an antigen recognizing construct of the invention comprises the respective CDR1 to CDR3 of one individual herein disclosed TCR variable region of the invention (see table 1 herein below and the example section).

The term "specificity" or "antigen specificity" or "specific for" a given antigen, as used herein means that the ABP can specifically bind to said antigen, preferably a RAC1 and/or RAC2 derived antigen, more preferably with high avidity, when said antigen is presented by HLA, preferably by HLA-A*02. For example, a TCR, as antigen recognizing construct, may be considered to have "antigenic specificity" for the RAC₂^{P29L} and/or RAC1^{P29S} antigenic peptide, if T-cells expressing the TCR, or an antigen-binding variant or derivative, and contacted with a RAC₂^{P29L} and/or RAC1^{P29S} derived antigenic peptide presenting HLA secrete at least about 200 pg/ml or more (e.g., 250 pg/ml or more, 300 pg/ml or more, 400 pg/ml or more, 500 pg/ml or more, 600 pg/ml or more, 700 pg/ml or more, 1000 pg ml or more, 2,000 pg/ml or more, 2,500 pg/ml or more, 5,000 pg/ml or more) of interferon γ (IFN-γ) upon co-culture with target cells pulsed with a low concentration of an antigenic peptide, such as the RAC₂^{P29L} and/or RAC1^{P29S} epitopes and antigens (comprising the P29 mutated amino acid position) provided herein below (e.g., about 10⁻¹¹ mol/l, 10⁻¹⁰ mol/l, 10⁻⁹ mol/l, 10⁻⁸ mol/l, 10⁻⁷ mol/l, 10⁻⁶ mol/l, 10⁻⁵ mol/l). Alternatively, or additionally, a TCR may be considered to have "antigenic specificity" for the antigenic peptide, if T-cells expressing the TCR secrete at least twice as much IFN-γ as the untransduced background level of IFN-γ upon co-culture with target cells pulsed with a low concentration of the RAC₂^{P29L} and/or RAC1^{P29S} antigenic peptides. Such a "specificity" as described above can - for example - be analyzed with an ELISA.

In one alternative or additional embodiment of the invention, the antigen recognizing construct selectively binds to an antigenic peptide; preferably wherein the antigenic peptide is a protein epitope or peptide having an amino acid sequence shown in SEQ ID NO: 17 or 18, and preferably is a peptide consisting of the amino acid sequence shown in SEQ ID NO: 17 or 18, or a variant thereof, wherein the variant is an amino acid deletion, addition, insertion or substitution of not more than three, preferably two and most preferably not more than one amino acid position. Such detection is realized in context of an MHC presentation of the above peptides.

The term "selectivity" or "selective recognizing/binding" is understood to refer to the property of an ABP of the invention, such as a TCR or antibody, to selectively recognize or bind to preferably only one specific epitope and preferably shows no or substantially no cross-reactivity to another epitope. Preferably "selectivity" or "selective recognizing/binding" means that the antigen recognizing construct (e.g. a TCR) selectively recognizes or binds to preferably only one specific epitope and preferably shows no or substantially no cross-reactivity to another epitope, wherein said epitope is unique for one protein, such that the antigen recognizing construct shows no or substantially no cross-reactivity to another epitope and another protein. Surprisingly it was recognized that the preferred TCR of the invention show compared to prior art TCR an unexpected range of selectivity by recognizing both RAC1 and RAC2 neoepitopes, and have otherwise a reduced off-target binding to other epitope sequences - therefore the TCR of the invention are particularly useful for treating diseases associated with the expression of mutated RAC proteins, preferably RAC2^{P29L} and/or RAC1^{P29S}.

The ABP according to the invention is preferably selected from an antibody, or derivative or fragment thereof, or a T-cell receptor (TCR), or derivative or fragment thereof. A derivative or fragment of an antibody or TCR of the invention shall preferably retain the antigen binding/recognizing ability of the parent molecule, in particular its specificity and/or selectivity as explained above. Such binding functionality may be retained by the presence of a CDR3 region as defined herein. Such antigen binding specificity for a TCR is usually dependent on the HLA (MHC) presented antigenic peptide, so that the TCR forms a complex together with the peptide and the HLA molecule presenting the peptide.

In an embodiment of the invention, the inventive TCRs are able to recognize RAC2^{P29L} and/or RAC1^{P29S} antigens in a major histocompatibility complex (MHC) class I-dependent manner. "MHC class I-dependent manner," as used herein, means that the TCR elicits an immune response upon binding to RAC2^{P29L} and/or RAC1^{P29S} antigens within the context of an MHC class I molecule. The MHC class I molecule can be any MHC class I molecule known in the art, e.g., HLA-A molecules. In a preferred embodiment of the invention, the MHC class I molecule is an HLA-A^{∗}02 molecule.

The invention provides both single chain ABPs and double chain ABPs. The ABPs of the invention may be soluble or membrane bound constructs.

In an embodiment, the TCR alpha variable domain has at least one mutation relative to a TCR alpha domain shown in Table 1; and/or the TCR beta variable domain has at least one mutation relative to a TCR alpha domain shown in Table 1. In an embodiment, a TCR comprising at least one mutation in the TCR alpha variable domain and/or TCR beta variable domain has a binding affinity for, and/or a binding half-life for, a RAC2^{P29L} and/or RAC1^{P29S} peptide-HLA molecule complex, which is at least double that of a TCR comprising the unmutated TCR alpha domain and/or unmutated TCR beta variable domain.

The TCR alpha chains of the present description may further comprise a TCR alpha transmembrane domain and/or a TCR alpha intracellular domain. The TCR beta chains of the present description may further comprise a TCR beta transmembrane domain and/or a TCR beta intracellular domain.

The invention in particular provides a TCR as antigen recognizing construct, or fragment or derivative thereof. The TCR preferably is of human, which is understood as being generated from a human TCR locus and therefore comprising human TCR sequences. Furthermore, the TCR of the invention may be characterized in that it is of human origin and specifically recognizes a RAC2^{P29L} and/or RAC1^{P29S} antigen of the invention.

Another embodiment of the invention additionally or alternatively provides the antigen recognizing construct described above, which induces an immune response, preferably wherein the immune response is characterized by an increase in interferon (IFN) γlevels.

TCRs of the invention may be provided as single chain α or β, or γ and δ, molecules, or alternatively as double chain constructs composed of both the α and β chain, or γ and δ chain.

The antigen recognizing construct of the invention may comprise a TCR α or γ chain; and/or a TCR β or δ chain; wherein the TCR α or γ chain comprises a CDR3 having at least 50%, 60%, 70%, 80%, 90%, 95%, 98%, 99%, or 100% sequence identity to an amino acid sequence selected from SEQ ID NOs. 3, or 11, and/or wherein the TCR β or δ chain comprises a CDR3 having at least 50%, 60%, 70%, 80%, 90%, 95%, 98%, 99%, or 100% sequence identity to an amino acid sequence selected from SEQ ID NOs. 7 or 15. Also preferred is a ABP comprising a TCR α or γ chain; and/or a TCR β or δ chain; wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID NOs: 3 and 11; and/or wherein the TCR β or δ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID NOs: 7 and 15.

Most preferably, in some additional embodiments, wherein the disclosure refers to antigen recognizing constructs comprising any one, two or all of the CDR1 to CDR3 regions of the herein disclosed TCR chains (see table 1), such antigen recognizing constructs may be preferred, which comprise the respective CDR sequence of the invention with not more than three, two, and preferably only one, modified amino acid residues. A modified amino acid residue may be selected from an amino acid insertion, deletion or substitution. Most preferred is that the three, two, preferably only one modified amino acid residue is the first or last amino acid residue of the respective CDR sequence. If the modification is a substitution, then it is preferable in some embodiments that the substitution is a conservative amino acid substitution. Furthermore, it is preferred that any mutation in a CDR is in some embodiments either a mutation at the most C-terminal and/or most N-terminal end of such CDR.

If the antigen recognizing construct of the invention is composed of at least two amino acid chains, such as a double chain TCR, or antigen binding fragment thereof, the antigen recognizing construct may comprises in a first polypeptide chain the amino acid sequence according to SEQ ID NO: 3, and in a second polypeptide chain the amino acid sequence according to SEQ ID NO: 7; or in a first polypeptide chain the amino acid sequence according to SEQ ID NO: 11, and in a second polypeptide chain the amino acid sequence according to SEQ ID NO: 15.

Any one of the aforementioned double chain TCR, or antigen binding fragments thereof, are preferred TCR of the present invention. In some embodiments, the CDR3 of the double chain TCR of the invention may be mutated. Mutations of the CDR3 sequences as shown in table 1 as provided above preferably include a substitution, deletion, addition, or insertion of not more than three, preferably two, and most preferably not more than one amino acid residue. In some embodiments, the first polypeptide chain may be a TCR α or γ chain, and the second polypeptide chain may be a TCR β or δ chain. Preferred is the combination of an αβ or γδ TCR. As described such mutated CDR3 may in preferred embodiments include only conservative amino acid exchanges or deletions, and/or may be introduced at the most C-terminal and/or most N-terminal end of such CDR3.

The TCR, or the antigen binding fragment thereof, is in some embodiments composed of a TCR α and a TCR β chain, or γ and δ chain. Such a double chain TCR comprises within each chain variable regions, and the variable regions each comprise one CDR1, one CDR2 and one CDR3 sequence. The TCRs comprises the CDR1 to CDR3 sequences as comprised in the variable chain amino acid sequence of SEQ ID NO: 4 and SEQ ID NO: 8, or SEQ ID NO: 12 and SEQ ID NO: 16.

Some embodiments of the invention pertain to a TCR, or a fragment thereof, composed of a TCR α and a TCR β chain, wherein said TCR comprises the variable region sequences having at least 50%, 60%, 70%, 80%, 90%, 95%, 98%, 99%, or preferably 100% sequence identity to the amino acid sequence selected from the α and β chain according to SEQ ID NO: 4 and 8 respectively, or 11 and 16 respectively.

The inventive TCRs may further comprise a constant region derived from any suitable species, such as any mammal, e.g., human, rat, monkey, rabbit, donkey, or mouse. In an embodiment of the invention, the inventive TCRs further comprise a human constant region. In some preferred embodiments, the constant region of the TCR of the invention may be slightly modified, for example, by the introduction of heterologous sequences, preferably mouse sequences, which may increase TCR expression and stability.

The TCR α or γ chain of the invention may further comprise a CDR1 having at least 50%, 60%, 70%, 80%, 90%, 95%, 98%, 99%, or 100% sequence identity to, or having no more than three or two, preferably no more than one amino acid substitution(s), deletion(s) or insertion(s) compared to, a sequence selected from SEQ ID NOs: 1 and 9; and/or a CDR2 having an amino acid sequence with at least 80% sequence identity to, or having no more than three or two, preferably no more than one amino acid substitution(s), deletion(s) or insertion(s) compared to, a sequence selected from SEQ ID NOs: 2 and 10.

According to the invention the TCR β or δ chain may further comprise a CDR1 having at least 50%, 60%, 70%, 80%, 90%, 95%, 98%, 99%, or 100% sequence identity to, or having no more than three or two, preferably no more than one amino acid substitution(s), deletion(s) or insertion(s) compared to, a sequence selected from SEQ ID NOs: 5 and 13; and/or a CDR2 having an amino acid sequence with at least 80% sequence identity to, or having no more than three or two, preferably no more than one amino acid substitution(s), deletion(s) or insertion(s) compared to, a sequence selected from SEQ ID NOs: 6 and 14.

The antigen recognizing construct may in a further embodiment comprise a binding fragment of a TCR, and wherein said binding fragment comprises CDR1 to CDR3, optionally selected from the CDR1 to CDR3 sequences having the amino acid sequences of SEQ ID NOs. 1, 2, 3, or 5, 6, 7 or 9, 10, 11, or 13, 14, 15, each CDR independently having no more than three or two, preferably no more than one amino acid substitution(s), deletion(s) or insertion(s) compared to these sequences.

In further embodiments of the invention the antigen recognizing construct as described herein elsewhere is a TCR, or a fragment thereof, composed of at least one TCR α and one TCR β chain sequence, wherein said TCR α chain sequence comprises the CDR1 to CDR3 sequences having the amino acid sequences of SEQ ID NO: 1 to 3, and said TCR β chain sequence comprises the CDR1 to CDR3 sequences having the amino acid sequences of SEQ ID NO: 5 to 7; or wherein said TCR α chain sequence comprises the CDR1 to CDR3 sequences having the amino acid sequences of SEQ ID NO: 9 to 11, and said TCR β chain sequence comprises the CDR1 to CDR3 sequences having the amino acid sequences of SEQ ID NO: 13 to 15; each CDR independently having no more than three or two, preferably no more than one amino acid substitution(s), deletion(s) or insertion(s) compared to these sequences.

In further embodiments of the invention the antigen recognizing construct as described herein before is a TCR, or a fragment thereof, comprising at least one TCR α and one TCR β chain sequence, wherein said TCR α chain sequence comprises a variable region sequence having the amino acid sequence of SEQ ID NO: 4, and wherein said TCR β chain sequence comprises a variable region sequence having the amino acid sequence of SEQ ID NO: 8; or wherein said TCR α chain sequence comprises a variable region sequence having the amino acid sequence of SEQ ID NO: 12, and wherein said TCR β chain sequence comprises a variable region sequence having the amino acid sequence of SEQ ID NO: 16; each variable domain sequence having no more than 20, preferably no more than 15, more preferably no more than 10, 5, three or two, preferably no more than one amino acid substitution(s), deletion(s) or insertion(s) compared to these sequences.

In further embodiments of the invention the antigen recognizing construct as described herein before is a TCR, or a fragment thereof, further comprising a TCR constant region having at least 50%, 60%, 70%, 80%, 90%, 95%, 98%, 99%, or 100% sequence identity to an amino acid sequence selected from a human TCR constant domain.

As used herein, the term "murine" or "human," when referring to an antigen recognizing construct, or a TCR, or any component of a TCR described herein (e.g., complementarity determining region (CDR), variable region, constant region, α chain, and/or β chain), means a TCR (or component thereof), which is derived from a mouse or a human unrearranged TCR locus, respectively.

In an embodiment of the invention, chimeric TCR are provided, wherein the TCR chains comprise sequences from multiple species. Preferably, a TCR of the invention may comprise an α chain comprising a human variable region of an α chain and, for example, a murine constant region of a murine TCR α chain.

In one embodiment, the TCR of the invention is a human TCR comprising human variable regions according to the above embodiments and human constant regions.

In some embodiments the antigen recognizing construct is murinized or humanized. These terms are used when amino acid sequences from a foreign species are introduced into a construct of the invention.

The TCR of the invention may be provided as a single chain TCR (scTCR). A scTCR can comprise a polypeptide of a variable region of a first TCR chain (e.g., an alpha chain) and a polypeptide of an entire (full-length) second TCR chain (e.g., a beta chain), or vice ver-sa. Furthermore, the scTCR can optionally comprise one or more linkers which join the two or more polypeptides together. The linker can be, for instance, a peptide, which joins together two single chains, as described herein. Also provided is such a scTCR of the invention, which is fused to a human cytokine, such as IL-2, IL-7 or IL-15.

The ABP according to the invention can also be provided in the form of a multimeric complex, comprising at least two TCR or scTCR molecules, wherein said TCR or scTCR molecules are each fused to at least one biotin moiety, or other interconnecting molecule/linker, and wherein said TCR or scTCRs are interconnected by biotin-streptavidin interaction to allow the formation of said multimeric complex. Similar approaches known in the art for the generation of multimeric TCR are also possible and included in this disclosure. Also provided are multimeric complexes of a higher order, comprising more than two scTCR of the invention.

For the purposes of the present invention, a TCR is a moiety having at least one TCR alpha or gamma and/or TCR beta or delta variable domain. Generally, they comprise both a TCR alpha variable domain and a TCR beta variable domain, alternatively both a TCR gamma variable domain and a TCR delta variable domain. They may be αβ/γδ hetero-dimers or may be in single chain format. For use in adoptive therapy, an αβ or γδ heterodimeric TCR may, for example, be transfected as full-length chains having both cytoplasmic and transmembrane domains. If desired, an introduced disulfide bond between residues of the respective constant domains may be present.

In a preferred embodiment, the ABP of the invention is a human TCR, or fragment or derivative thereof. A human TCR or fragment or derivative thereof is a TCR, which comprises over 50% of the corresponding human TCR sequence. Preferably, only a small part of the TCR sequence is of artificial origin or derived from other species. It is known, however, that chimeric TCRs, e.g. derived from human origin with murine sequences in the constant domains, are advantageous. Particularly preferred are, therefore, TCRs in accordance with the present invention, which contains murine sequences in the extracellular part of their constant domains.

Thus, it is also preferred that the inventive ABP is able to recognize its respective antigen in a human leucocyte antigen (HLA) dependent manner, preferably in a HLA-A02 dependent manner. The term "HLA dependent manner" in the context of the present invention means that the antigen recognizing construct binds to the antigen only in the event that the antigenic peptide is presented by said HLA.

The antigen recognizing construct in accordance with the invention in one embodiment preferably induces an immune response, preferably wherein the immune response is characterized by the increase in interferon (IFN) γ levels.

Also provided by the invention is a polypeptide comprising a functional portion of any of the TCRs (or functional variants thereof) described herein, for examples, of any one of the TCRs selected from the TCR as provided in the example section and table 1. The term "polypeptide" as used herein includes oligopeptides and refers to a single chain of amino acids connected by one or more peptide bonds. With respect to the inventive polypeptides, the functional portion can be any portion comprising contiguous amino acids of the TCR (or functional variant thereof), of which it is a part, provided that the functional portion specifically binds to the RAC1/RAC2 neoantigen, preferably as disclosed herein as SEQ ID NOs: 17 and 18. The term "functional portion" when used in reference to a TCR (or functional variant thereof) refers to any part or fragment of the TCR (or functional variant thereof) of the invention, which part or fragment retains the biological activity of the TCR (or functional variant thereof), of which it is a part (the parent TCR or parent functional variant thereof). Functional portions encompass, for example, those parts of a TCR (or functional variant thereof) that retain the ability to specifically bind to the RAC1/RAC2 antigen (in an HLA dependent manner), or detect, treat, or prevent a RAC1/RAC2 associated disorder, such as cancer, to a similar extent, the same extent, or to a higher extent, as the parent TCR (or functional variant thereof). In reference to the parent TCR (or functional variant thereof), the functional portion can comprise, for instance, about 10%, 25%, 30%, 50%, 68%, 80%, 90%, 95%, or more, of the parent TCR variable sequences (or functional variant thereof).

The functional portion can comprise additional amino acids at the amino or carboxy terminus of the portion, or at both termini, in which additional amino acids are not found in the amino acid sequence of the parent TCR or functional variant thereof. Desirably, the additional amino acids do not interfere with the biological function of the functional portion, e.g., specifically binding to the RAC1/RAC2 antigens; and/or having the ability to detect cancer, treat or prevent cancer, etc. More desirably, the additional amino acids enhance the biological activity, as compared to the biological activity of the parent TCR or functional variant thereof.

The polypeptide can comprise a functional portion of either or both of the α and β chains of the TCRs or functional variant thereof of the invention, such as a functional portion comprising one of more of CDR1, CDR2, and (preferably) CDR3 of the variable region(s) of the α chain and/or β chain of a TCR or functional variant thereof of the invention. In an embodiment of the invention, the polypeptide can comprise a functional portion comprising the amino acid sequence of SEQ ID NO: 3, 7, 11, or 15 (CDR3 of the variable regions of the TCR of the invention), or a combination thereof. In an embodiment of the invention, the inventive polypeptide can comprise, for instance, the variable region of the inventive TCR or functional variant thereof comprising a combination of the CDR regions set forth above. In this regard, the polypeptide can comprise the amino acid sequence of any of SEQ ID NO: 4, 8, 12, and 16 (the variable regions of an α or β chain of the TCR of the invention).

In some instances, the construct of the invention may comprise one or two polypeptide chains comprising a sequence, or a sequence encoded by a nucleic acid sequence, according to any one of the SEQ ID NO: 1 to 16 (CDR sequences and variable region sequences), or functional fragments thereof, and further comprise(s) other amino acid sequences, e.g., an amino acid sequence encoding an immunoglobulin or a portion thereof, then the inventive protein can be a fusion protein. In this regard, the invention also provides a fusion protein comprising at least one of the inventive polypeptides described herein along with at least one other polypeptide. The other polypeptide can exist as a separate polypeptide of the fusion protein, or can exist as a polypeptide, which is expressed in frame (in tandem) with one of the inventive polypeptides described herein. The other polypeptide may include any peptidic or proteinaceous molecule, or a portion thereof, including, but not limited to an immunoglobulin, CD3, CD4, CD8, an MHC molecule, a CD1 molecule, e.g., CD1a, CDib, CD1c, CD1d, etc.

The fusion protein can comprise one or more copies of the inventive polypeptide and/or one or more copies of the other polypeptide. For instance, the fusion protein can comprise 1, 2, 3, 4, 5, or more, copies of the inventive polypeptide and/or of the other polypeptide. Suitable methods of making fusion proteins are known in the art, and include, for example, recombinant methods. In some embodiments of the invention, the TCRs (and functional portions and functional variants thereof), polypeptides, and proteins of the invention may be expressed as a single protein comprising a linker peptide linking the α chain and the β chain, and linking the γ chain and the δ chain. In this regard, the TCRs (and functional variants and functional portions thereof), polypeptides, and proteins of the invention comprising the amino acid sequences of the variable regions of the TCR of the invention and may further comprise a linker peptide. The linker peptide may advantageously facilitate the expression of a recombinant TCR (including functional portions and functional variants thereof), polypeptide, and/or protein in a host cell. The linker peptide may comprise any suitable amino acid sequence. Linker sequences for single chain TCR constructs are well known in the art. Such a single chain construct may further comprise one, or two, constant domain sequences. Upon expression of the construct including the linker peptide by a host cell, the linker peptide may also be cleaved, resulting in separated α and β chains, and separated γ and δ chain.

As already mentioned above, the binding functionality of the TCR of the invention may be provided in the framework of an antibody. For example, CDR sequences of the TCR of the invention, possibly including additional 3, 2 or 1N and/or c terminal framework residues, may be directly grafted into an antibody variable heavy/light chain sequence. The term "antibody" in its various grammatical forms is used herein to refer to immunoglobulin molecules and immunologically active portions of immunoglobulin molecules, i.e., molecules that contain an antigen-binding site or a paratope. Such molecules are also referred to as "antigen binding fragments" of immunoglobulin molecules. The invention further provides an antibody, or antigen binding portion thereof, which specifically binds to the antigens described herein. The antibody can be any type of immunoglobulin that is known in the art. For instance, the antibody can be of any isotype, e.g., IgA, IgD, IgE, IgG, IgM, etc. The antibody can be monoclonal or polyclonal. The antibody can be a naturally-occurring antibody, e.g., an antibody isolated and/or purified from a mammal, e.g., mouse, rabbit, goat, horse, chicken, hamster, human, etc. Alternatively, the antibody can be a genetically-engineered antibody, e.g., a humanized antibody or a chimeric antibody. The antibody can be in monomeric or polymeric form.

The term "antibody" includes, but is not limited to, genetically engineered or otherwise modified forms of immunoglobulins, such as intrabodies, chimeric antibodies, fully human antibodies, humanized antibodies (e.g. generated by "CDR-grafting"), antibody fragments, and heteroconjugate antibodies (e.g., bispecific antibodies, diabodies, triabodies, tetra-bodies, etc.). The term "antibody" includes cys-diabodies and minibodies. Thus, each and every embodiment provided herein in regard to "antibodies", or "antibody like constructs" is also envisioned as, bispecific antibodies, diabodies, scFv fragments, chimeric antibody receptor (CAR) constructs, diabody and/or minibody embodiments, unless explicitly denoted otherwise. The term "antibody" includes a polypeptide of the immunoglobulin family or a polypeptide comprising fragments of an immunoglobulin that is capable of non-covalently, reversibly, and in a specific manner binding a corresponding antigen, preferably a RAC1/RAC2 mutated antigenic peptide of the invention, as disclosed herein. An exemplary antibody structural unit comprises a tetramer. In some embodiments, a full-length antibody can be composed of two identical pairs of polypeptide chains, each pair having one "light" and one "heavy" chain (connected through a disulfide bond). Antibody structure and isotypes are well known to the skilled artisan (for example from Janeway's Immuno-biology, 9th edition, 2016).

The recognized immunoglobulin genes of mammals include the kappa, lambda, alpha, gamma, delta, epsilon, and mu constant region genes, as well as the myriad immuno-globulin variable region genes (for more information on immunoglobulin genes see the international ImMunoGeneTics information system^{®}, Lefranc M-P et al, Nucleic Acids Res. 2015 Jan;43(Database issue):D413-22; and http://www.imgt.org/). For full-length chains, the light chains are classified as either kappa or lambda. For full-length chains, the heavy chains are classified as gamma, mu, alpha, delta, or epsilon, which in turn de-fine the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (VL) and variable heavy chain (VH) refer to these regions of light and heavy chains respectively. As used in this invention, an "antibody" encompasses all variations of antibody and fragments thereof. Thus, within the scope of this concept are full length antibodies, chimeric antibodies, humanized antibodies, single chain antibodies (scFv), Fab, Fab', and multimeric versions of these fragments (e.g., F(ab')2) with the same, essentially the same or similar binding specificity. In some embodiments, the antibody binds specifically to a mutated RAC1/RAC2 peptide of the invention. Preferred antigen recognizing constructs according to the invention include an antibody heavy chain, preferably the variable domain thereof, or an antigen binding fragment thereof, and/or an antibody light chain, preferably the variable domain thereof, or an antigen binding fragment thereof. Similarly, disulfide-stabilized variable region fragments (dsFv) can be prepared by recombinant DNA technology, antibody fragments of the invention, however, are not limited to these exemplary types of antibody fragments. Also, the antibody, or antigen binding portion thereof, can be modified to comprise a detectable label, such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles). In some instances, the TCR CDR3 sequence may be slightly modified, but preferably by not more than 3 amino acid residues, preferably only two and most preferably only one amino acid position, as compared to the CDR3 sequences provided in SEQ ID NOs: 3, 7, 11, and 15. Preferably, the antibodies comprise the CDR3, preferably all of CDR1 to CDR3 regions in the combination, as indicated for the TCR of the invention in table 1, in each case independently, optionally with not more than three or two, preferably one, amino acid substitution(s), insertion(s) and/or deletion(s) compared to these sequences.

Suitable methods of making antibodies are known in the art. For instance, standard hybridoma methods are described in, e.g., Kohler and Milstein, Eur. J. Immunol, 5, 51 1-519 (1976), Harlow and Lane (eds.), Antibodies: A Laboratory Manual, CSH Press (1988), and C.A. Janeway et al. (eds.), Immunobiology, 8 Ed., Garland Publishing, New York, NY (2011)). Alternatively, other methods, such as EBV-hybridoma methods (Haskard and Archer, J. Immunol. Methods, 74(2), 361-67 (1984), and Roder et al, Methods Enzymol, 121, 140-67 (1986)), and bacteriophage vector expression systems (see, e.g., Huse et al., Science, 246,1275-81 (1989)) are known in the art. Further, methods of producing anti-bodies in non-human animals are described in, e.g., U.S. Patents 5,545,806, 5,569,825, and 5,714,352, and U.S. Patent Application Publication No. 2002/0197266.

Some embodiments of the invention also pertain to TCRs, or functional fragments and polypeptides thereof, which are soluble TCRs. As used herein, the term "soluble T-cell receptor" refers to heterodimeric truncated variants of native TCRs, which comprise extracellular portions of the TCR α-chain and β-chain, for example linked by a disulfide bond, but which lack the transmembrane and cytosolic domains of the native protein. The terms "soluble T-cell receptor α-chain sequence and soluble T-cell receptor β-chain sequence" refer to TCR α-chain and β-chain sequences that lack the transmembrane and cytosolic domains. The sequence (amino acid or nucleic acid) of the soluble TCR α-chain and β-chains may be identical to the corresponding sequences in a native TCR or may comprise variant soluble TCR α-chain and β-chain sequences, as compared to the corresponding native TCR sequences. The term "soluble T-cell receptor" as used herein encompasses soluble TCRs with variant or non-variant soluble TCR α-chain and β-chain sequences. The variations may be in the variable or constant regions of the soluble TCR α-chain and β-chain sequences and can include, but are not limited to, amino acid deletion, insertion, substitution mutations as well as changes to the nucleic acid sequence, which do not alter the amino acid sequence. Soluble TCR of the invention in any case retain the binding functionality of their parent molecules.

In **a second aspect,** the invention pertains a nucleic acid, or a nucleic acid construct (NAC), having a nucleic acid sequence encoding for an ABP of the invention.

The above problem is further solved by a nucleic acid encoding for an antigen recognizing construct or ABP of the invention, or any of the aforementioned protein or polypeptide constructs. The nucleic acid preferably (a) has a strand encoding for an antigen recognizing construct according to the invention; (b) has a strand complementary to the strand in (a); or (c) has a strand that hybridizes under stringent conditions with a molecule as described in (a) or (b). Stringent conditions are known to the person of skill in the art, specifically from Sambrook et al, "Molecular Cloning". In addition to that, the nucleic acid optionally has further sequences, which are necessary for expressing the nucleic acid sequence corresponding to the protein, specifically for expression in a mammalian/human cell. The nucleic acid used can be contained in a vector suitable for allowing expression of the nucleic acid sequence corresponding to the peptide in a cell. However, the nucleic acids can also be used to transform an antigen-presenting cell, which may not be restricted to classical antigen-presenting cells, such as dendritic cells, in such a way that they themselves produce the corresponding proteins on their cellular surface.

In some embodiments, the polypeptides of the antigen recognizing constructs or ABP can be encoded by nucleic acids and expressed in vivo or in vitro. Thus, in some embodiments, a nucleic acid encoding an antigen recognizing construct or ABP is provided. In some embodiments, the nucleic acid encodes one part or monomer of an antigen recognizing construct or ABP of the invention (for example one of two chains of a TCR of the invention), and/or another nucleic acid encodes another part or monomer of an antigen recognizing con-struct or ABP of the invention (for example the other of two chains of the TCR). In some embodiments, the nucleic acid encodes two or more antigen recognizing construct or ABP polypeptide chains, for example, at least 2 TCR chains. Nucleic acids encoding multiple antigen recognizing construct or ABP chains can include nucleic acid cleavage sites between at least two chain sequences, can encode transcription or translation start site between two or more chains sequences, and/or can encode proteolytic target sites between two or more antigen recognizing construct or ABP chains.

By "nucleic acid" as used herein includes "polynucleotide," "oligonucleotide," and "nucleic acid molecule," and generally means a polymer of DNA or RNA, which can be single-stranded or double-stranded, synthesized or obtained (e.g., isolated and/or purified) from natural sources, which can contain natural, non-natural or altered nucleotides, and can contain a natural, non-natural or altered internucleotide linkage, such as a phosphoroamidate linkage or a phosphorothioate linkage, instead of the phosphodiester found between the nucleotides of an unmodified oligonucleotide.

Preferably, the nucleic acids of the invention are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be in vitro replication or in vivo replication. The nucleic acid can comprise any nucleotide sequence, which encodes any of the TCRs, polypeptides, or proteins, or functional portions or functional variants thereof described herein.

In **a third aspect,** the invention pertains to a recombinant vector comprising the nucleic acid of the invention.

Furthermore, the invention provides a vector comprising a nucleic acid in accordance to the invention as described above. Desirably, the vector is an expression vector or a recombinant expression vector. The term "recombinant expression vector" refers in context of the present invention to a nucleic acid construct that allows for the expression of an mRNA, protein or polypeptide in a suitable host cell. The recombinant expression vector of the invention can be any suitable recombinant expression vector, and can be used to transform or transfect any suitable host. Suitable vectors include those designed for propagation and expansion or for expression or both, such as plasmids and viruses. Examples of animal expression vectors include pEUK-Cl, pMAM, and pMAMneo. Preferably, the recombinant expression vector is a viral vector, e.g., a retroviral vector. The recombinant expression vector comprises regulatory sequences, such as transcription and translation initiation and termination codons, which are specific to the type of host cell (e.g., bacterium, fungus, plant, or animal), into which the vector is to be introduced and in which the expression of the nucleic acid of the invention may be performed. Furthermore, the vector of the invention may include one or more marker genes, which allow for selection of transformed or transfected hosts. The recombinant expression vector can comprise a native or normative promoter operably linked to the nucleotide sequence encoding the constructs of the invention, or to the nucleotide sequence, which is complementary to or which hybridizes to the nucleotide sequence encoding the constructs of the invention. The selections of promoters include, e.g., strong, weak, inducible, tissue-specific and developmental-specific promoters. The promoter can be a non-viral promoter or a viral promoter. The inventive recombinant expression vectors can be designed for either transient expression, for stable expression, or for both. Also, the recombinant expression vectors can be made for constitutive expression or for inducible expression.

Such vectors can either contain nucleic acids encoding one or both TCR chains, therefore may comprise a sequence encoding for the alpha and/or beta chain of the ABP of the invention.

In **a fourth aspect,** the invention pertains to a recombinant host cell, comprising or expressing an ABP of the invention, or comprising a nucleic acid or vector of the invention.

The invention also pertains to a host cell comprising an antigen recognizing construct or ABP in accordance with the invention. Specifically, the host cell of the invention comprises a nucleic acid, or a vector as described herein above. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing a recombinant TCR, polypeptide, or protein, the host cell is preferably a mammalian cell. Most preferably, the host cell is a human cell. While the host cell can be of any cell type, can originate from any type of tissue, and can be of any developmental stage, the host cell preferably is a peripheral blood leukocyte (PBL) or a peripheral blood mononuclear cell (PBMC). More preferably, the host cell is a T-cell. The T-cell can be any T-cell, such as a cultured T-cell, e.g., a primary T-cell, or a T-cell from a cultured T-cell line, e.g., Jurkat, SupTi, etc., or a T-cell obtained from a mammal, preferably a T-cell or T-cell precursor from a human patient. If obtained from a mammal, the T-cell can be obtained from numerous sources, including but not limited to blood, bone marrow, lymph node, the thymus, or other tissues or fluids. T-cells can also be enriched for or purified. Preferably, the T-cell is a human T-cell. More preferably, the T-cell is a T-cell isolated from a human. The T-cell can be any type of T-cell and can be of any developmental stage, including but not limited to, CD4-positive and/or CD8-positive, CD4-positive helper T-cells, e.g., Th1 and Th2 cells, CD8-positive T-cells (e.g., cytotoxic T-cells), tumor infiltrating cells (TILs), memory T-cells, naive T-cells, and the like. Preferably, the T-cell is a CD8-positive T-cell or a CD4-positive T-cell.

Preferably, the host cell of the invention is a lymphocyte, preferably, a T lymphocyte, such as a CD4-positive or CD8-positive T-cell. The host cell furthermore preferably is a RAC2^{P29L} and/or RAC1^{P29S} antigen reactive T-cell specific for RAC2^{P29L} and/or RAC1^{P29S} antigen presenting cells.

In **a fifth aspect,** the invention pertains to a pharmaceutical composition comprising one or more, preferably a combination of, an ABP of the invention, or a nucleic acid or cell of the invention.

Thus also provided is a pharmaceutical composition, comprising any of the herein described products of the invention and TCR materials of the invention, specifically any proteins, nucleic acids or host cells. In a preferred embodiment the pharmaceutical composition is for immune therapy, preferably adoptive cell therapy.

Preferably, the inventive TCR material is administered by injection, e.g., intravenously. When the inventive TCR material is a host cell expressing the inventive TCR (or functional variant thereof), the pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment, the pharmaceutically acceptable carrier is supplemented with human serum albumen.

For purposes of the invention, the amount or dose (e.g., numbers of cells when the inventive TCR material is one or more cells) of the inventive TCR material administered may be sufficient to affect, e.g., a therapeutic or prophylactic response, in the subject or animal over a reasonable time frame. For example, the dose of the inventive TCR material should be sufficient to bind to a cancer antigen, or detect, treat or prevent cancer in a period of from about 2 hours or longer, e.g., 12 to 24 or more hours, from the time of administration. In certain embodiments, the time period could be even longer. The dose will be determined by the efficacy of the particular inventive TCR material and the condition of the animal (e.g., human), as well as the body weight of the animal (e.g., human) to be treated.

It is contemplated that the inventive pharmaceutical compositions, antigen recognizing constructs, TCRs (including functional variants thereof), polypeptides, proteins, nucleic acids, recombinant expression vectors, host cells, or populations of cells can be used in methods of treating or preventing a cancer, or condition associated with a RAC2^{P29L} and/or RAC1^{P29S} -positive cells. The inventive TCRs (and functional variants thereof) are believed to bind specifically to the RAC2^{P29L} and/or RAC1^{P29S} antigens of the invention, such that the TCR (or related inventive polypeptide or protein and functional variants thereof), when expressed by or on a cell, such as a T-cell, is able to mediate an immune response against a target cell expressing the RAC2^{P29L} and/or RAC1^{P29S} antigen of the invention, preferably presenting RAC2^{P29L} and/or RAC1^{P29S} antigenic peptides via MHC I on the surface of said target cell. In this regard, the invention provides a method of treating or preventing a condition, in particular an cancer disease, in a mammalian subject, comprising administering to the mammal any of the pharmaceutical compositions, isolated ABPs, or combinations of ABPs of the invention, in particular TCRs (and functional variants thereof), polypeptides, or proteins described herein, any nucleic acid or recombinant expression vector comprising a nucleotide sequence encoding any of the TCRs (and functional variants thereof), polypeptides, proteins described herein, or any host cell or population of cells comprising a nucleic acid or recombinant vector, which encodes any of the constructs of the invention (and functional variants thereof), polypeptides, or proteins described herein, in an amount effective to treat or prevent the condition in the mammal, wherein the condition is preferably a cancer positive for a mutated RAC1 and/or RAC2 or any disease or condition associated with the expression of such mutated proteins.

Examples of pharmaceutically acceptable carriers or diluents useful in the present invention include stabilizers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

In **a sixth aspect,** the invention pertains to a compound or composition comprising any of the products of the invention, or combinations thereof, for use in medicine, in particular for use in the treatment of a disease associated with a mutated RAC1/RAC2 antigen in a subject.

The constructs, proteins, TCRs antibodies, polypeptides and nucleic acids of the invention are in particular for use in immune therapy, preferably, in adoptive T-cell therapy. The administration of the compounds of the invention can, for example, involve the infusion of T-cells of the invention into said patient. Preferably, such T-cells are autologous T-cells of the patient and in vitro transduced with a nucleic acid or antigen recognizing construct of the present invention. Hence, as used herein, the term "adoptive immunotherapy" or "adoptive cell therapy" (ACT) refers to a process whereby autologous or allogeneic cells of various hematopoietic lineages (e.g., lymphocytes or T-cells) are transferred to a patient or subject to treat disease. The term "adoptive T-cell therapy" refers to a process whereby autologous or allogeneic T-cells are transferred to a patient or subject to treat disease, preferably such T-cells recombinantly express the TCR of the invention (the ABP of the invention).

Compounds and compositions of the invention are useful in the prevention or treatment of proliferative disorders such as cancer. The terms "cancer" or "tumor" or "proliferative disorder" refer to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within an animal, or may be a non-tumorigenic cancer cell, such as a leukemia cell. Cancers include, but are not limited to, B cell cancer, e.g., multiple myeloma, Waldenstroem's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, and the like. Other non-limiting examples of types of cancers applicable to the methods encompassed by the present invention include human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, the cancer whose phenotype is determined by the method of the invention is an epithelial cancer such as, but not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (e.g., serous ovarian carcinoma), or breast carcinoma. The epithelial cancers may be characterized in various other ways including, but not limited to, serous, endometrioid, mucinous, clear cell, brenner, or undifferentiated. In some embodiments, the present invention is used in the treatment, diagnosis, and/or prognosis of lymphoma or its subtypes, including, but not limited to, lymphocyte-rich classical Hodgkin lymphoma, mixed cellularity classical Hodgkin lymphoma, lymphocyte-depleted classical Hodgkin lymphoma, nodular sclerosis classical Hodgkin lymphoma, anaplastic large cell lymphoma, diffuse large B-cell lymphomas, MLL+ pre B-cell ALL) based upon analysis of markers described herein.

Preferred cancers of the present invention are those which are characterized by the expression of RAC1 and/or RAC2, and in preferred embodiments by the expression of a mutated variant of RAC1 and/or RAC2, namely preferably RAC1^{P29S} and/or RAC2^{P29L}, at least such cancers which are characterized by a direct or immune cell mediated presentation of peptides derived from RAC1/RAC2, preferably peptides comprising the mutated amino acid P29S or P29L, and most preferably a peptide according to SEQ ID NO: 17 or 18.

Most preferably, an ABP of the invention is for use in the treatment of a cancer disease, wherein the cancer disease is characterized by the expression of a mutated RAC1 protein, preferably a RAC1^{P29S}. In the examples it was surprisingly shown that the TCR of the invention raised against RAC2 derived mutated peptides (RAC2^{P29L}) have a significantly better therapeutic effect in vivo against RAC1 driven cancers, compared to TCR which were generated with RAC1 peptides. In accordance, the TCR of the invention are particularly useful for treatments or prevention of diseases characterized by the expression of RAC1^{P29s}.

The inventive antigen recognizing constructs, ABPs, such as TCRs, polypeptides, proteins (including functional variants thereof), nucleic acids, recombinant expression vectors, host cells (including populations thereof), and antibodies (including antigen binding portions thereof), all of which are collectively referred to as "inventive TCR materials" hereinafter, can be formulated into a composition, such as a pharmaceutical composition. In this regard, the invention provides a pharmaceutical composition comprising any of the antigen recognizing constructs, TCRs, polypeptides, proteins, functional portions, functional variants, nucleic acids, expression vectors, host cells (including populations thereof), and antibodies (including antigen binding portions thereof) described herein, and a pharmaceutically acceptable carrier, excipient and/or stabilizer. The inventive pharmaceutical compositions containing any of the inventive TCR materials can comprise more than one inventive TCR material, e.g., a polypeptide and a nucleic acid, or two or more different TCRs (including functional portions and functional variants thereof). Alternatively, the pharmaceutical composition can comprise an inventive TCR material in combination with another pharmaceutically active agent(s) or drug(s), such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. Preferably, the carrier is a pharmaceutically acceptable carrier. With respect to pharmaceutical compositions, the carrier can be any of those conventionally used for the particular inventive TCR material under consideration. Such pharmaceutically acceptable carriers are well-known to those skilled in the art and are readily available to the public. It is preferred that the pharmaceutically acceptable carrier be one, which has no detrimental side effects or toxicity under the conditions of use.

As used herein, the term "subject" refers to a human or another mammal (e.g., primate, dog, cat, goat, horse, pig, mouse, rat, rabbit, and the like), that preferably suffers from a disease treatable or preventable in accordance with the herein described invention. Such subject is preferably a subject positive for a peptide presentation of a RAC peptide as disclosed herein.

The term "treatment" is used herein to characterize a method or process that is aimed at (1) delaying or preventing the onset of a disease or condition (e.g., cancer); (2) slowing down or stopping the progression, aggravation, or deterioration of the symptoms of the disease or condition; (3) bringing about amelioration of the symptoms of the disease or condition; or (4) curing the disease or condition. A treatment may be administered prior to the onset of the disease or condition, for a prophylactic or preventing action. Alternatively, or additionally, a treatment may be administered after initiation of the disease, for a therapeutic action.

In **a seventh aspect,** the invention pertains to a method of manufacturing a RAC1 and/or 2 neoantigen specific antigen recognizing construct expressing cell line, comprising
- providing a suitable host cell,
- providing a genetic construct comprising a coding sequence encoding the ABP of the invention,
- introducing into said suitable host cell said genetic construct,
- expressing said genetic construct by said suitable host cell.

The method may further comprise a step of cell surface presentation of said antigen recognizing construct on said suitable host cell.

In other preferred embodiments, the genetic construct is an expression construct comprising a promoter sequence operably linked to said coding sequence.

Preferably, said antigen recognizing construct is of mammalian origin, preferably of human origin. The preferred suitable host cell for use in the method of the invention is a mammalian cell, such as a human cell, in particular a human T lymphocyte. T-cells for use in the invention are described in detail herein above.

Also encompassed by the invention are embodiments, wherein said antigen recognizing construct is a modified TCR, wherein said modification is the addition of functional domains, such as a label or a therapeutically active substance. Furthermore, encompassed are TCR having alternative domains, such as an alternative membrane anchor domain instead of the endogenous transmembrane region.

Desirably, the transfection system for introducing the genetic construct into said suitable host cell is a retroviral vector system. Such systems are well known to the skilled artisan.

Also comprised by the present invention is in one embodiment the additional method steps of isolation and purification of the antigen recognizing construct from the cell and, optionally, the reconstitution of the translated antigen recognizing construct-fragments in a T-cell.

In an alternative aspect of the invention a T-cell is provided obtained or obtainable by a method for the manufacture of a T-cell receptor (TCR) producing cell line as described, which TCR is specific for RAC1/RAC2 positive cells, preferably cells positive for RAC2^{P29L} and/or RAC1^{P29s}, and has high avidity as described herein above. Such a T-cell is depending on the host cell used in the method of the invention, for example, a human or non-human T-cell, preferably a human TCR.

Another aspect of the invention further pertains to a method for detecting a RAC2^{P29L} and/or RAC1^{P29S} antigen, or a complex of MHC and the RAC2^{P29L} and/or RAC1^{P29S} antigenic peptide (protein epitope of the RAC2^{P29L} and/or RAC1^{P29S} associated protein), in a (biological) sample -such as one obtained from a subject or patient - comprising contacting the sample with an ABP specifically binding to said RAC2^{P29L} and/or RAC1^{P29S} peptide presented on an infected cell, or to the RAC2^{P29L} and/or RAC1^{P29S} peptide/MHC complex, and detecting the binding between said ABP and said RAC2^{P29L} and/or RAC1^{P29S} peptide, or to the RAC2^{P29L} and/or RAC1^{P29S} peptide/MHC complex. In some embodiments, the antigen recognizing construct or ABP is a TCR or antibody, or similar constructs, or preferably the antigen recognizing construct according to the herein described invention. In some embodiments, the (biological) sample is a sample of a liver (such as one of those described elsewhere herein) for example a sample comprising possible RAC2^{P29L} and/or RAC1^{P29S} positive cells of a subject, such as melanoma cells.

Also provided is a method of treating cancer in a subject in need thereof, comprising:
- isolating a cell from said subject;
- transforming the cell with at least one vector encoding an antigen recognizing construct or ABP of the present invention to produce a transformed cell;
- expanding the transformed cell to produce a plurality of transformed cells; and
- administering the plurality of transformed cells to said subject.

Also provided is a method of treating cancer in a subject in need thereof, comprising:
- isolating a cell from a healthy donor;
- Transforming the cell with a vector encoding an antigen recognizing construct of the present invention to produce a transformed cell;
- expanding the transformed cell to produce a plurality of transformed cells; and
- administering the plurality of transformed cells to said subject.

Also provided is a method of detecting a RAC2^{P29L} and/or RAC1^{P29S} positive cancer cell in a biological sample comprising:
- contacting the biological sample with an ABP of the present description, wherein the biological sample preferably comprises host cells positive for RAC2^{P29L} and/or RAC1^{P29S};
- detecting binding of the ABP to the biological sample.

In some embodiments, the method of detecting a cell positive for RAC2^{P29L} and/or RAC1^{P29S} is carried out in vitro, in vivo or in situ, for example using a sample of a subject comprising cells associated with the disease and potentially comprising RAC2^{P29L} and/or RAC1^{P29S}.

Also provided is a method of detecting the presence of a condition in a mammal. The method comprises (i) contacting a sample comprising one or more cells from the mammal with any of the inventive TCRs (and functional variants thereof), polypeptides, proteins, nucleic acids, recombinant expression vectors, host cells, populations of cells, antibodies, or antigen binding portions thereof, or pharmaceutical compositions described herein, thereby forming a complex, and detecting the complex, wherein detection of the complex is indicative of the presence of the condition in the mammal, wherein the condition is cancer, such as a cancer positive for RAC2^{P29L} and/or RAC1^{P29S}.

With respect to the inventive method of detecting a condition in a mammal, the sample of cells can be a sample comprising whole cells, lysates thereof, or a fraction of the whole cell lysates, e.g., a nuclear or cytoplasmic fraction, a whole protein fraction, or a nucleic acid fraction.

For purposes of the inventive detecting method, the contacting can take place in vitro or in vivo with respect to the mammal. Preferably, the contacting is in vitro.

Also, detection of the complex can occur through any number of ways known in the art. For instance, the inventive antigen recognizing constructs (and functional variants thereof), polypeptides, proteins, nucleic acids, recombinant expression vectors, host cells, populations of cells, or antibodies or TCRs, or antigen binding portions thereof, described herein, can be labeled with a detectable label such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles).

For purposes of the inventive methods, wherein host cells or populations of cells are administered, the cells can be cells that are allogeneic or autologous to the mammal. Preferably, the cells are autologous to the mammal.

In general, the invention provides a method for treating a subject suffering from a RAC2^{P29L} and/or RAC1^{P29S} positive cancer comprising the administration of the antigen recognizing constructs, ABPs, nucleic acids, vectors, pharmaceutical compositions and/or host cell as disclosed by the present invention. Preferably the subject is a subject in need of such a treatment. The subject in preferred embodiments is a mammalian subject, preferably a human patient, suffering from a RAC2^{P29L} and/or RAC1^{P29S} positive disease.

In addition to the above, the present invention further pertains to the following itemized embodiments:
Item 1: An isolated antigen binding protein (ABP) which specifically binds to a RAC1 and/or RAC2, preferably mutated RAC1 and/or RAC2, derived antigenic peptide, or a variant thereof, and wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 3, 5, 11, and 15.
Item 2:The isolated ABP of item 1, wherein said ABP is capable of specifically and/or selectively binding to an antigenic peptide derived from RAC2^{P29L} and/or RAC1^{P29S}, and preferably wherein such derived peptide comprises the RAC2^{P29L} and/or RAC1^{P29S} mutation, therefore, the P29L and/or P29S mutation. The position of the mutation is less relevant. Most preferably the isolated ABP of item 1 binds to a peptide of SEQ ID NO: 17 and/or 18.
Item 3:The isolated ABP of item 1 or 2, wherein the ABP is an antibody, or an antigen binding derivative or fragment thereof, or, preferably, is a T cell receptor (TCR), or an antigen binding derivative or fragment thereof.
Item 4:The isolated ABP of any one of items 1 to 3, wherein said ABP binds to a human leucocyte antigen (HLA) presented antigenic peptide, wherein said HLA is preferably type A2, preferably wherein the ABP forms a specific complex with the MHC and the antigenic peptide.
Item 5:The isolated ABP of any one of items 1 to 4, wherein the ABP specifically and/or selectively binds to an epitope having the amino acid sequence selected from an MHC presented peptide of a RAC2^{P29L} and/or RAC1^{P29S} protein, preferably the peptide comprises such mutated amino acid position, and most preferably a peptide consisting of SEQ ID NO: 17 or 18.
Item 6:The isolated ABP of any one of items 1 to 5, wherein the ABP is an α/β-TCR, or antigen binding derivative or fragment thereof, or the construct is a γ/δ-TCR, or antigen binding derivative or fragment thereof.
Item 7:The isolated ABP of any one of items 1 to 6, characterized in that the ABP is of human origin and specifically and/or selectively recognizes an RAC2^{P29L} and/or RAC1^{P29S} antigenic peptide.
Item 8: The isolated ABP of any one of items 1 to 7, wherein said ABP is capable of inducing an immune response in a subject, optionally wherein the immune response is characterized by an increase in interferon (IFN) γ levels.
Item 9:The isolated ABP of any one of items 1 to 8, comprising a TCR α or γ chain; and/or a TCR β or δ chain; wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 3, and 11; and/or wherein the TCR β or δ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 7, and 15.
Item 10: The isolated ABP of item 9, wherein the TCR α or γ chain further comprises a CDR1 having an amino acid sequence with at least 80% sequence identity to, or having no more than three or two, preferably no more than one amino acid substitution(s), deletion(s) or insertion(s) compared to, a sequence selected from SEQ ID Nos: 1 or 9; and/or a CDR2 having an amino acid sequence with at least 80% sequence identity to, or having no more than three or two, preferably no more than one amino acid substitution(s), deletion(s) or insertion(s) compared to, a sequence selected from SEQ ID Nos: 2, or 10.
Item 11: The isolated ABP of item 9 or 10, wherein the TCR β or δ chain further comprises a CDR1 having an amino acid sequence with at least 80% sequence identity to, or having no more than three or two, preferably no more than one amino acid substitution(s), deletion(s) or insertion(s) compared to, a sequence selected from SEQ ID Nos: 5, and 13; and/or a an amino acid sequence with at least 80% sequence identity to, or having no more than CDR2 having three or two, preferably no more than one amino acid substitution(s), deletion(s) or insertion(s) compared to, a sequence selected from SEQ ID Nos: 6 and 14.
Item 12: The isolated ABP of any one of items 1 to 11, comprising a TCR variable chain region having at least 80% sequence identity to, or having no more than 20, preferably no more than 15, more preferably no more than 10, 5, three or two, preferably no more than one amino acid substitution(s), deletion(s) or insertion(s) compared to, an amino acid sequence selected from SEQ ID Nos. 4, 8,12, and 16.
Item 13: The isolated ABP of any one of 1 to 12, wherein the construct is humanized, chimerized and/or murinized.
Item 14: The isolated ABP of any one of items 1 to 13, comprising a binding fragment of a TCR, and wherein said binding fragment comprises CDR1 to CDR3, optionally selected from the CDR1 to CDR3 sequences having the amino acid sequences of SEQ ID Nos. 1, 2, 3, or 5, 6, 7 or 9, 10, 11, or 13 14, 15; each CDR independently having no more than three or two, preferably no more than one amino acid substitution(s), deletion(s) or insertion(s) compared to these sequences.
Item 15: The isolated ABP of any one of items 1 to 14, wherein the construct is a TCR, or a fragment thereof, composed of at least one TCR α and one TCR β chain sequence, wherein said TCR α chain sequence comprises the CDR1 to CDR3 sequences having the amino acid sequences of SEQ ID NO: 1 to 3, and said TCR β chain sequence comprises the CDR1 to CDR3 sequences having the amino acid sequences of SEQ ID NO: 5 to 7; or wherein said TCR α chain sequence comprises the CDR1 to CDR3 sequences having the amino acid sequences of SEQ ID NO: 9 to 11, and said TCR β chain sequence comprises the CDR1 to CDR3 sequences having the amino acid sequences of SEQ ID NO: 13 to 15; each CDR independently having no more than three or two, preferably no more than one amino acid substitution(s), deletion(s) or insertion(s) compared to these sequences.
Item 16: The isolated ABP of any one of items 1 to 15, wherein the ABP is a TCR, or an antigen-binding fragment or derivative thereof, comprising at least one TCR α and one TCR β chain sequence, wherein said TCR α chain sequence comprises a variable region sequence having the amino acid sequence of SEQ ID No. 4, and wherein said TCR β chain sequence comprises a variable region sequence having the amino acid sequence of SEQ ID No. 8; or wherein said TCR α chain sequence comprises a variable region sequence having the amino acid sequence of SEQ ID No. 12, and wherein said TCR β chain sequence comprises a variable region sequence having the amino acid sequence of SEQ ID No. 16; each variable domain sequence having no more than 20, preferably no more than 15, more preferably no more than 10, 5, three or two, preferably no more than one amino acid substitution(s), deletion(s) or insertion(s) compared to these sequences.
Item 17: The isolated ABP of any one of items 1 to 16, wherein the ABP is a TCR, or an antigen-binding fragment or derivative thereof, further comprising a TCR constant region, preferably a human TCR constant region sequence.
Item 18: An isolated nucleic acid encoding for an ABP of any one of items 1 to 17.
Item 19: The isolated nucleic acid of item 18, comprising a nucleic acid sequence having at least 80% sequence identity to, or having no more than 20, preferably no more than 15, more preferably no more than 10, 5, three or two, preferably no more than one nucleic acid substitution(s), deletion(s) or insertion(s) compared to, a nucleic acid sequence encoding for a polypeptide selected from SEQ ID Nos 4, 8, 12, and 16.
Item 20: A recombinant vector comprising a nucleic acid of item 18 or 19.
Item 21: A recombinant host cell comprising an ABP of any one of items 1 to 17, or a nucleic acid of item 18 or 19, or a vector of item 20.
Item 22: The recombinant host cell of item 21, wherein the cell is a lymphocyte, preferably a T lymphocyte or T lymphocyte progenitor, more preferably a CD4 or, most preferably a CD8 positive T-cell.
Item 23: A pharmaceutical composition comprising the ABP of any one of items 1 to 17, or a nucleic acid of item 18 or 19, or a vector of item 20, or the host cell of item 21 or 22, and a pharmaceutical acceptable carrier, stabilizer and/or excipient; preferably, wherein the pharmaceutical composition comprises at least two different ABP of any one of items 1 to 17.
Item 24: A compound or composition for use in medicine, wherein the compound or composition is selected from the ABP of any one of items 1 to 17, or a nucleic acid of item 18 or 19, or a vector of item 20, or the host cell of item 21 or 22, or the pharmaceutical composition of item 23.
Item 25: The compound or composition for use of item 24, wherein the use is for a treatment of a proliferative disorder, preferably cancer, such as a cancer positive for an expression of RAC2^{P29L} and/or RAC1^{P29S}.
Item 26: The compound or composition for use of item 25, wherein the treatment comprises immune therapy, preferably adoptive autologous or heterologous T-cell therapy.
Item 27: The compound or composition for use of item 26, wherein the T-cell therapy involves the use of cell comprising the ABP or nucleic acid of any one of the preceding items.
Item 28: A method of manufacturing a RAC2^{P29L} and/or RAC1^{P29S} specific antigen recognizing construct expressing cell line, comprising
   (a) providing a suitable host cell,
   (b) providing a genetic construct comprising a coding sequence encoding the ABP according to any of items 1 to 17,
   (c) introducing into said suitable host cell said genetic construct,
   (d) expressing said genetic construct by said suitable host cell.
Item 29: The method of item 28, further comprising cell surface presentation of said antigen recognizing construct.
Item 30: The method of item 28 or 29, wherein the genetic construct is an expression construct comprising a promoter sequence operably linked to said coding sequence.
Item 31: The method of any one of items 28 to 30, wherein said antigen recognizing construct is of mammalian origin, preferably of human origin.
Item 32: The method of any one of items 28 to 31, wherein said suitable host cell is a mammalian cell, optionally selected from a human cell or a human T lymphocyte.
Item 33: The method of any one of items 28 to 32, wherein said antigen recognizing construct is a modified TCR, wherein said modification comprises addition of a functional domain comprising a label, or an alternative domain comprising a membrane anchor domain.
Item 34: The method of item 33 wherein said antigen recognizing construct is an ABP, preferably is an alpha/beta TCR, gamma/delta TCR, or a TCR variant such as a single chain TCR (scTCR).
Item 35: The method of any one of items 28 to 34, wherein said genetic construct is introduced into said suitable host cell by retroviral transfection.
Item 36: The method of any one of items 28 to 35, further comprising the isolation and purification of the antigen recognizing construct from the suitable host cell and, optionally, reconstitution of the antigen recognizing construct in a T-cell.
Item 37: The method of any one of items 28 to 35, wherein the suitable host cell is a cell, such as a T-cell, obtained from a patient suffering from a disorder associated with RAC2^{P29L} and/or RAC1^{P29S} expression, such as preferably a cancer.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1****:** shows identification and isolation of RAC2P29L-specific CTLs in ABabDII mice.

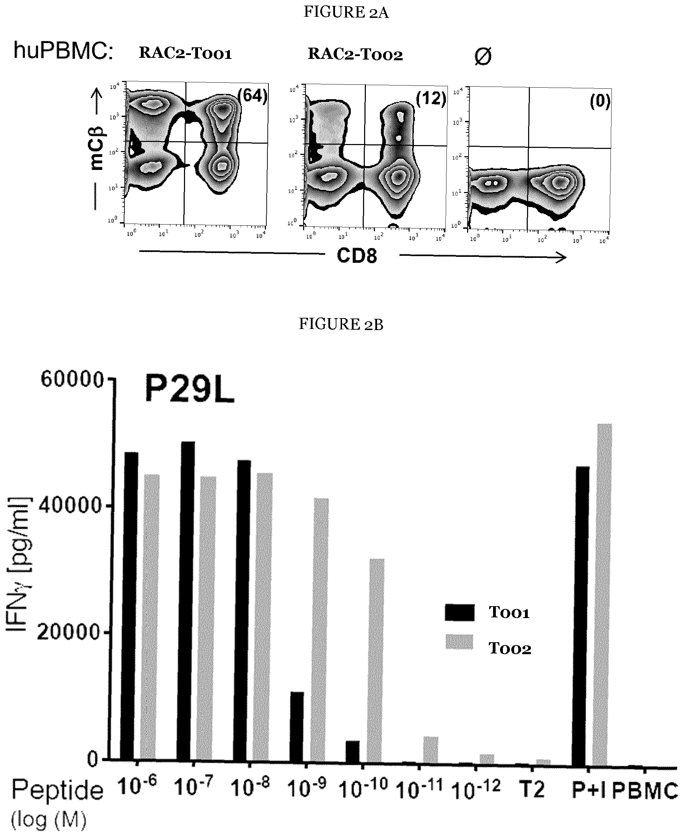

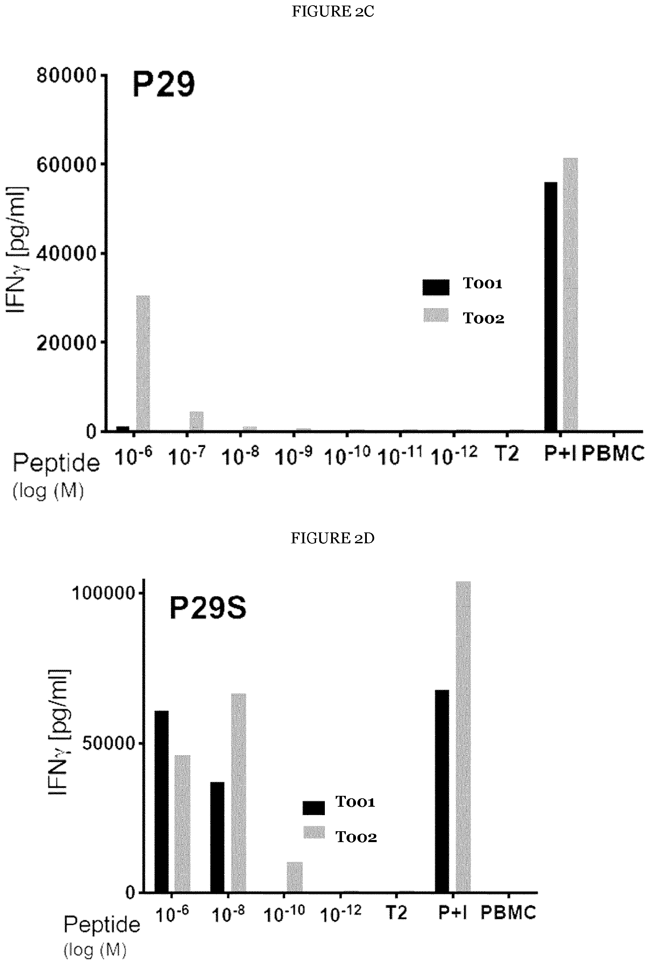

**Figure 2****:** shows TCR gene transfer confers specificity for mutant RAC2^{P29L} peptide.

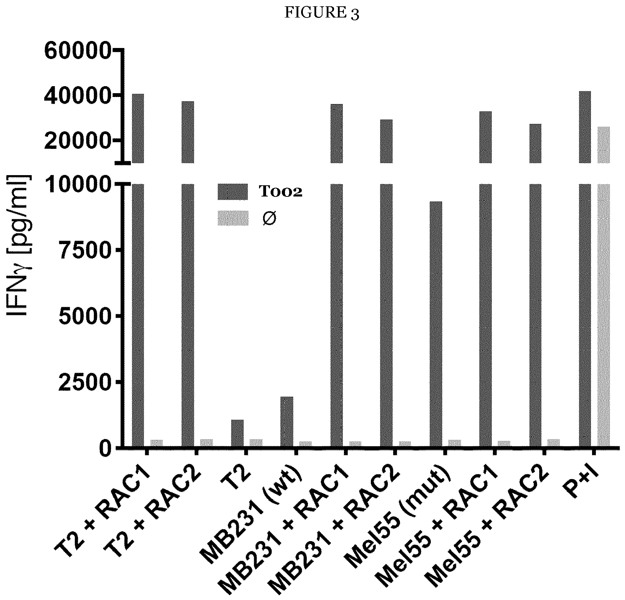

**Figure 3****:** shows recognition of human melanoma cells harboring RAC1^{P29S} by RAC2P29LTCR-redirected T cells.
**Figure 4****:** shows heterologous RAC2P29L-specificTCRs elicits efficient tumor rejection upon ATT.

**SEQ ID NO: 1** to 16: show TCR sequences of the invention disclosed herein in table 1 below.

**SEQ ID NO:** 17 shows the peptide RAC2^{P29L}: FLGEYIPTV

**SEQ ID NO:** 18 shows the peptide RAC1^{P29S}: FSGEYIPTV

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### The examples show:

### Example 1: Generation of TCR of the invention

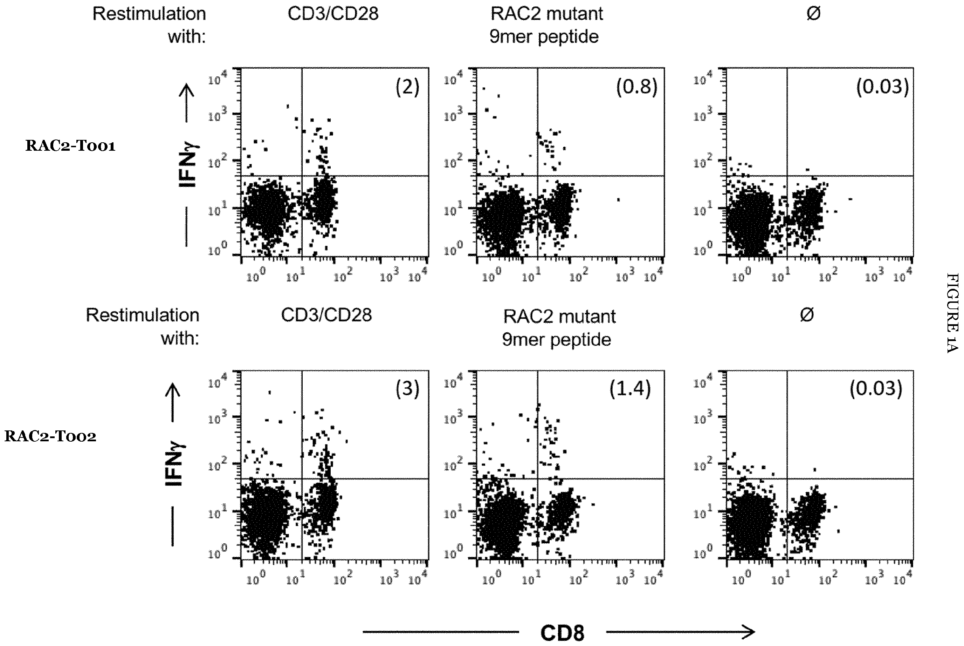

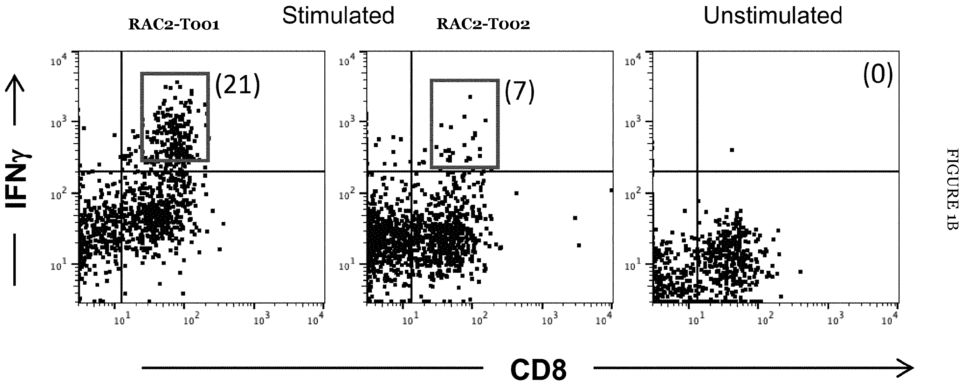

Figure 1: Identification and isolation of RAC2^{P29L}-specific CTLs in ABabDII mice. Immunization with mutant RAC2^{P29L} peptide induces CTL response in ABabDII mice. Representative example of ex vivo ICS analysis of RAC2 mutant peptide immunized ABabDII mice 7 days after the last immunization. Cells are gated on lymphocytes and CD3+ cells. Numbers in brackets represent percent IFNg+ CD8+ T cells. (B) Identification of IFNγ+ CD8+ T cells using IFNγ-capture assay. A representative IFNγ capture ab/CD8+ staining of mutant RAC2^{P29L} specific CD8+ T cells 10 days after spleen cell culture in the presence of 10-8 M mutant RAC2 peptide. Cells are gated on lymphocytes and CD3+ cells, unstimulated splenocytes served as negative control. Numbers in brackets represent percent IFNγ++ CD8+ T cells. Sorted cells are depicted in Figure 1. Table 1 shows the TCR amino acid sequences as isolated.

**Table 1: Isolated TCR Sequences of the invention**

| **TCR Designation** | **TCR Chain** | **TCR Domain** | **Sequence** | **SEQ ID NO:** |
|---|---|---|---|---|
| RAC2-T001 | alpha | CDRA1 | DSASNY | **1** |
| RAC2-T001 | alpha | CDRA2 | IRSNVGE | **2** |
| RAC2-T001 | alpha | CDRA3 | CAASMGNAGNMLTF | **3** |
| RAC2-T001 | alpha | Variable Domain A | MTSIRAV FIFLWLQLDL VNGENVEQHP STLSVQEGDS AVIKCTYSDS ASNYFPWYKQ ELGKGPQLII DIRSNVGEKK DQRIAVTLNK TAKHFSLHIT ETQPEDSAVY FCAASMGNAG NMLTFGGGTR LMVKPH | **4** |
| RAC2-T001 | beta | CDRB1 | SNHLY | **5** |
| RAC2-T001 | beta | CDRB2 | FYNNEI | **6** |
| RAC2-T001 | beta | CDRB3 | CAS SETS GASYEQYF | **7** |
| RAC2-T001 | beta | Variable Domain B | MDTWLV CWAIFSLLKA GLTEPEVTQT PSHQVTQMGQ EVILRCVPIS NHLYFYWYRQ ILGQKVEFLV SFYNNEISEK SEIFDDQFSV ERPDGSNFTL KIRSTKLEDS AMYFCASSET SGASYEQYFG PGTRLTVT | **8** |
| RAC2-T002 | alpha | CDRA1 | SSVPPY | **9** |
| RAC2-T002 | alpha | CDRA2 | YTTGATLV | **10** |
| RAC2-T002 | alpha | CDRA3 | CAVGANNLFF | **11** |
| RAC2-T002 | alpha | Variable Domain A | MLLLLV PVLEVIFTLG GTRAQSVTQL GSHVSVSEGA LVLLRCNYSS SVPPYLFWYV QYPNQGLQLL LKYTTGATLV KGINGFEAEF KKSETSFHLT KPSAHMSDAA EYFCAVGANN LFFGTGTRLT VIPY | **12** |
| RAC2-T002 | beta | CDRB1 | SNHLY | **13** |
| RAC2-T002 | beta | CDRB2 | FYNNEI | **14** |
| RAC2-T002 | beta | CDRB3 | CASSEWRGSSYNEQFF | **15** |
| RAC2-T002 | beta | Variable Domain B | MDTWLV CWAIFSLLKA GLTEPEVTQT PSHQVTQMGQ EVILRCVPIS NHLYFYWYRQ ILGQKVEFLV SFYNNEISEK SEIFDDQFSV ERPDGSNFTL KIRSTKLEDS AMYFCASSEW RGSSYNEQFF GPGTRLTVL | **16** |

### Example 2: TCR gene transfer confers specificity for mutant RAC2^{P29L} peptide.

The corresponding TCR alpha and beta chains isolated from two RAC2^{P29L} peptide immunized ABabDII mice (RAC2-T001 and RAC2-T001) were cloned into retroviral vector pMP71 and reexpressed in human PBMC. (A)Transduction efficacy is measured by staining of the mouse TCRb chain on CD8+ T cells, number of positive CD8+ T cells is shown in brackets. (B-D) IFNγ-production of RAC2^{P29L} TCR-transduced T cells (RAC2-T002, black bars; RAC2-T001 TCR, red bars) upon coculture with peptide-loaded T2 cells. Either mutant (B) or wildtype (C) RAC2 peptide or RAC1^{P29S} peptide (D) was used for loading; as negative control T2 cells were not loaded (T2), for maximal stimulation PMA and ionomycin (P+I) were added to the coculture.

### Example 3: Recognition of human melanoma cells harboring RAC1^{P29S} by RAC2^{P29L} TCR-redirected T cells

Human PBMCs were retrovirally transduced with RAC2^{P29L}-specific RAC2-T002 TCR. IFNγ production of RAC2P29L- TCR-transduced T cells upon coculture with MB231 harboring RAC1/2 wt genes and Mel55 harboring RAC1^{P29S} gene is shown. As control mutant peptideloaded cells (+RAC1 or +RAC2) or no cells (ø) were used in the coculture. For maximal stimulation PMA and ionomycin (P+I) were added to the coculture.

### Example 4: Heterologous RAC2^{P29L}-specific TCRs elicits efficient tumor rejection upon ATT.

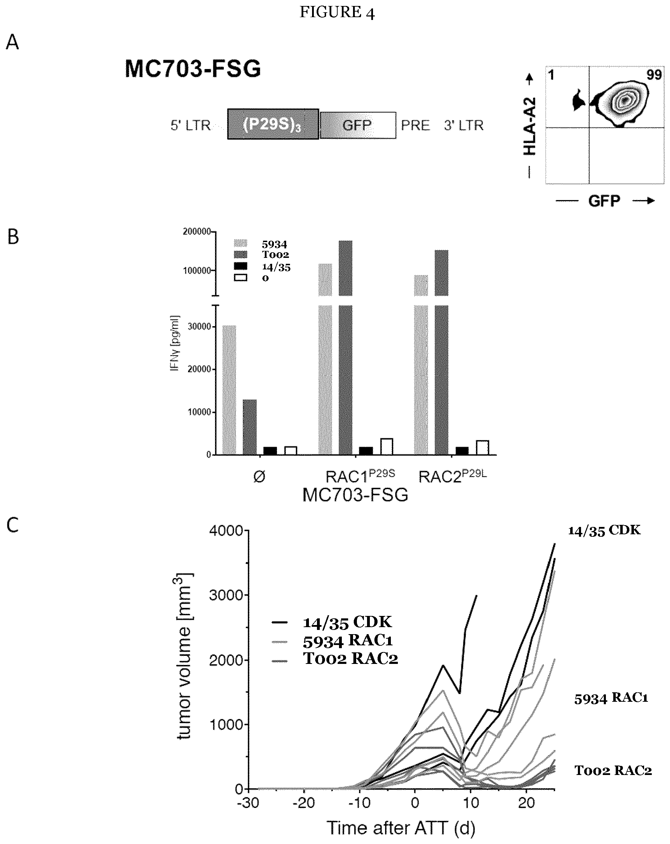

Figure 4 (A): for analysis of the in vivo efficacy of RAC1/2-specific TCRs HHD+ MC703 tumor cells that express minigenes encoding 3 copies of the RAC1^{P29S} epitope (MC703-FSG) were generated by retroviral transduction and subsequent FACsort for gfp+ cells, the amount of HLA-A2+gfp+ tumor cells used for subsequent experiments is shown. (B) Mouse T cells obtained from HHD transgenic mice were retrovirally transduced with RACi- and RAC2-specific TCRs.

Respective CD8+ T cells confer efficient recognition of epitope-expressing tumor cells, 14/35 TCR transduced HHD+ T cells and non-transduced HHD+ T cells (Ø) were used as negative controls. (C) HHD Rag-/- mice bearing established MC703-FSG tumors were treated with either 5934 RACi-specific or RAC2-T001 RAC2-specific T cells (adjusted to 1x106 CD8+TCR+-Te). Tumor-bearing HHD Rag-/- mice that received T cells expressing a CDK4R24C-specific TCR (14/35) are shown as control. One representative experiment is shown.

## Claims

1. **An isolated antigen binding protein (ABP) which specifically binds to a mutated RAC1 and/or RAC2 derived antigenic peptide, or to a variant thereof,** and wherein the isolated ABP comprises a T cell receptor (TCR) α or γ chain; and/or a TCR β or δ chain; wherein the TCR α or γ chain comprises a complementary determining region (CDR)3 having an amino acid sequence with at least 80% sequence identity to, or having no more than three amino acid substitution(s), deletion(s) or insertion(s) compared to, a sequence selected from SEQ ID Nos: 3 and 11; and/or wherein the TCR β or δ chain comprises a CDR3 having an amino acid sequence with at least 80% sequence identity to, or having no more than three amino acid substitution(s), deletion(s) or insertion(s) compared to, a sequence selected from SEQ ID Nos: 7 and 15.

2. The isolated ABP of claim 1, wherein said ABP is capable of specifically and/or selectively binding to an antigenic peptide comprising the P29 mutated amino acid position of RAC1 and/or RAC2, preferably the antigenic peptide of SEQ ID NO: 17 and/or 18.

3. The isolated ABP of claim 1 or 2, wherein the ABP is a TCR, or an antigen binding derivative or fragment thereof.

4. The isolated ABP of any one of claims 1 to 3, wherein the TCR α or γ chain further comprises a CDR1 having an amino acid sequence with at least 80% sequence identity to, or having no more than three amino acid substitution(s), deletion(s) or insertion(s) compared to, a sequence selected from SEQ ID Nos: 1 or 9; and/or a CDR2 having an amino acid sequence with at least 80% sequence identity to, or having no more than three amino acid substitution(s), deletion(s) or insertion(s) compared to, a sequence selected from SEQ ID Nos: 2, or 10.

5. The isolated ABP of any one of claims 1 to 4, wherein the TCR β or δ chain further comprises a CDR1 having an amino acid sequence with at least 80% sequence identity to, or having no more than three amino acid substitution(s), deletion(s) or insertion(s) compared to, a sequence selected from SEQ ID Nos: 5, and 13; and/or a an amino acid sequence with at least 80% sequence identity to, or having no more than three amino acid substitution(s), deletion(s) or insertion(s) compared to, a sequence selected from SEQ ID Nos: 6 and 14.

6. The isolated ABP of any one of claims 1 to 5, comprising a TCR variable chain region having at least 80% sequence identity to, or having no more than 20 amino acid substitution(s), deletion(s) or insertion(s) compared to, an amino acid sequence selected from SEQ ID Nos. 4, 8,12, and 16.

7. The isolated ABP of any one of claims 1 to 6, wherein the ABP is a TCR, or an antigenbinding fragment or derivative thereof, further comprising a TCR constant region, preferably a human TCR constant region sequence.

8. **An isolated nucleic acid** encoding for an ABP of any one of claims 1 to 7.

9. **A recombinant vector** comprising a nucleic acid of claim 8.

10. **A recombinant host cell** comprising an ABP of any one of claims 1 to 7, or a nucleic acid of claim 8, or a vector of claim 9.

11. The recombinant host cell of claim 10, wherein the cell is a lymphocyte, preferably a T lymphocyte or T lymphocyte progenitor, more preferably a CD4 or, most preferably, a CD8 positive T-cell.

12. **A pharmaceutical composition** comprising the ABP of any one of claims 1 to 7, or a nucleic acid of claim 8, or a vector of claim 9, or the host cell of claim 10 or 11, and a pharmaceutical acceptable carrier, stabilizer and/or excipient; preferably, wherein the pharmaceutical composition comprises at least two different ABP of any one of claims 1 to 7.

13. **A compound or composition for use in medicine,** wherein the compound or composition is selected from the ABP of any one of claims 1 to 7, or a nucleic acid of claim 8, or a vector of claim 9, or the recombinant host cell of claim 10 or 11, or the pharmaceutical composition of claim 12.

14. The compound or composition for use of claim 13, wherein the treatment comprises immune therapy, preferably adoptive, autologous or heterologous T-cell therapy, involving the use of said cell comprising the ABP or nucleic acid of any one of the preceding claims.

15. **A method of manufacturing a RAC2^{P29L} and/or RAC1^{P29S} specific antigen recognizing construct expressing cell line,** comprising
(a) providing a suitable host cell,
(b) providing a genetic construct comprising a coding sequence encoding the ABP according to any of claims 1 to 7,
(c) introducing into said suitable host cell said genetic construct,
(d) expressing said genetic construct by said suitable host cell to obtain the specific antigen recognizing construct expressing cell line.
